(19) Europäisches Patentamt
European Patent Office
Office européen des brevets

(11) EP 2 108 650 A1

# (12) EUROPEAN PATENT APPLICATION

(43) Date of publication:
**14.10.2009 Bulletin 2009/42**

(51) Int Cl.:
***C07D 407/04*** *(2006.01)* ***C07D 309/38*** *(2006.01)*

(21) Application number: **08007043.6**

(22) Date of filing: **09.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(83) **Declaration under Rule 32(1) EPC (expert solution)**

(71) Applicant: **Leibniz-Institut für Naturstoff-Forschung und Infektionsbiologie**
**07745 Jena (DE)**

(72) Inventors:
- **Hertweck, Christian**
  **04105 Leipzig (DE)**
- **Werneburg, Martina**
  **07745 Jena (DE)**
- **Roth, Martin**
  **07749 Jena (DE)**
- **Dahse, Hans-Martin**
  **99423 Weimar (DE)**

(74) Representative: **Forstmeyer, Dietmar et al**
**BOETERS & LIECK**
**Oberanger 32**
**80331 München (DE)**

## (54) Novel aureothin derivatives

(57) This invention relates to the field of biologically active compounds and specifically to novel aureothin derivatives, pharmaceutical compositions comprising these novel aureothin derivatives, methods for the production of the aureothin derivatives and their use as fungicide, antibiotic as well as antitumor agent.

Fig. 1

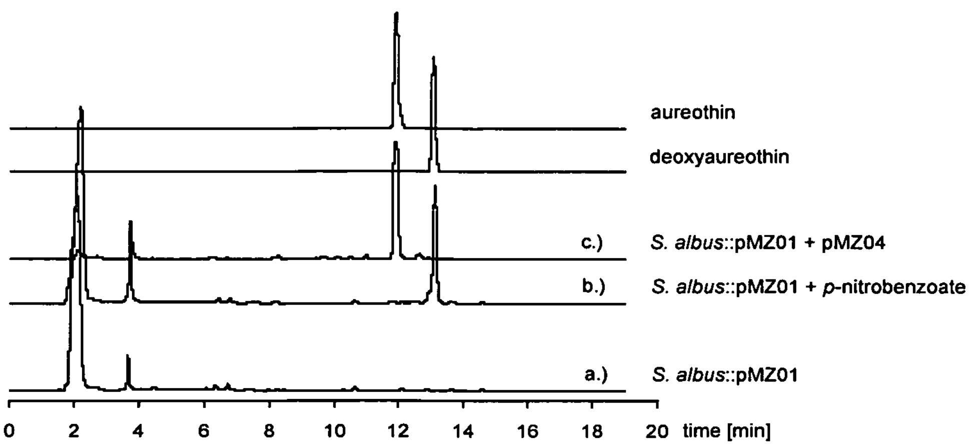


EP 2 108 650 A1

## Description

This invention relates to the field of biologically active compounds and specifically to novel aureothin derivatives, pharmaceutical compositions comprising these novel aureothin derivatives, methods for the production of the aureothin derivatives and their use as fungicide, antibiotic as well as antitumor agent.

Aureothin (formula **A**) presents a polyketide-shikimate hybrid from the soil bacterium *Streptomyces thioluteus* (K. Maeda, J. Antibiot. (Tokyo) 1953, 6, 137; Y. Hirata, H. Nakata, K. Yamada, K. Okuhara, T. Naito, Tetrahedron 1961, 14, 252).

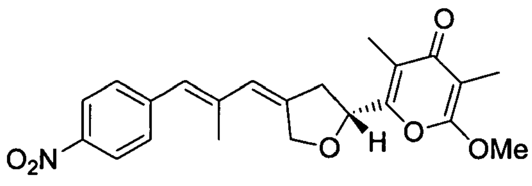

**(A)**

In addition to a variety of pharmacological properties including cytotoxic, antibacterial and antifungal activities aureothin offers some pecularities within its biosynthesis. Elucidation of the biosynthesis gene cluster (J. He, C. Hertweck, Chem. Biol. 2003, 10, 1225) revealed that within the formation of the starter unit p-nitrobenzoate (PNBA) a novel manganese dependent *N*-oxygenase AurF (J. He, C. Hertweck, J. Am. Chem. Soc. 2004, 126, 3694; R. Winkler, C. Hertweck, ChemBioChem 2007, 8, 973; G. Zocher, R. Winkler, C. Hertweck, G. E. Schulz, J. Mol. Biol. 2007) is involved (***Scheme 1***). In addition lacking a separate loading domain is striking with the conventional architecture of a type I polyketide synthase (PKS) in bacteria (J. Staunton, K. J. Weissman, Nat. Prod. Rep. 2001, 18, 380). Four modules AurABC catalyze the required elongation (either methyl-CoA or methyl-malonyl-CoA) and reduction cycles. Applying the first module AurA twice constitutes an exception to the principle of colinearity. Post-PKS enzymes like the methyltransferase Aurl effecting *O*-methylation of the pyrone ring followed by a cytochrome P450 monooxygenase AurH mediated introduction of the chiral tetrahydrofuran ring (J. He, M. Müller, C. Hertweck, J. Am. Chem. Soc. 2004, 126, 16742; M. Müller, J. He, C. Hertweck, ChemBioChem 2006, 7, 37) complete the aureothin biosynthesis (**Scheme 1**).

**Scheme 1** below shows a model of the aureothin biosynthesis. Targets for mutasynthesis are marked with arrows and include AurF for the synthesis of the starter unit PNBA and AurH within post-PKS processings for the introduction of the chiral THF ring. The precursor PNBA-CoA can be supplemented as R'=CoA (activation within biosynthesis) or R'=SNAC (synthetic activation).

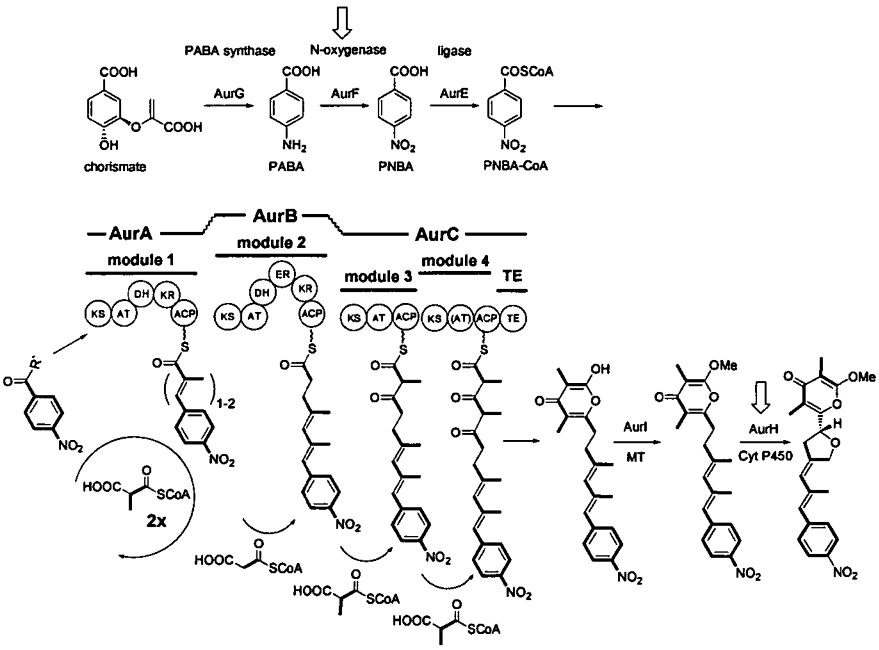


(Scheme 1)

On one hand, there is a rapid decline in the effectiveness of antibiotics and fungicides due to the emergence of resistance to many antibacterial and antifungal agents, respectively. Similarly, there hardly exist effective therapies for the treatment of cancer. On the other hand, the discovery of novel agents has become an increasing challenge. Still, microbial natural products are one of the most promising sources for novel antibiotics, fungicides as well as antitumor agents. This is, because natural products own an element of structural complexity which is required for the inhibition of many bacterial or fungal protein targets. Accordingly, there is a need for a constant supply of new antibiotics and fungicides for effective treatment of bacterial and fungal infections as well as for novel compounds having antitumor activity.

Therefore, the problem underlying the present invention is to provide novel compounds, namely aureothin derivatives having antibacterial, antifungal and/or antitumor activity.

The present invention relates to a compound, namely an aureothin derivative of the general formula (I) or (II):

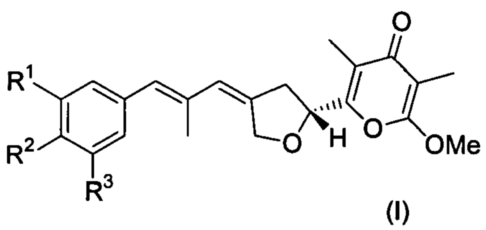
(I)

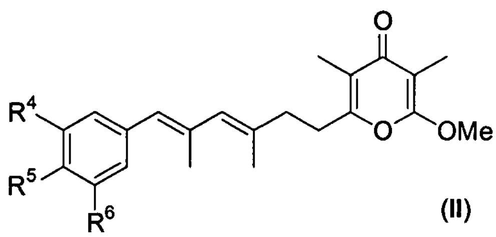
(II)

or a pharmacologically acceptable salt, solvate, hydrate or a pharmacologically acceptable formulation thereof, wherein

$R^1$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl,

alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^1$ is taken together with $R^2$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^2$ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^2$ is taken together with $R^3$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^3$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes;

$R^4$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^4$ is taken together with $R^5$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^5$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^5$ is taken together with $R^6$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^6$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino,

alkoxy, carboxyl, alkyl, alkynyl, alkenyl, aryl, sulfonyl, phosphoryl, anticalins, antibodies, folic acid, fluorescence dyes or labels, or molecular probes.

Compounds are generally described herein using standard nomenclature. For compounds having asymmetric centers, it should be understood that, unless otherwise specified, all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope, *i.e.*, an atom having the same atomic number but a different mass number. By way of general example, and without limitation, isotopes of hydrogen include tritium and deuterium and isotopes of carbon include $^{11}C$, $^{13}C$, and $^{14}C$.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example, a chiral HPLC column.

Compounds herein may also be described using a general formula that includes variables such as, e.g., A, $R^1$-$R^4$, Y, etc. Unless otherwise specified, each variable within such a formula is defined independently of any other variable, and any variable that occurs more than one time in a formula is defined independently at each occurrence. Thus, for example, if a group is shown to be substituted with 0-2 $R^*$, the group may be unsubstituted or substituted with up to two R groups and $R^*$ at each occurrence is selected independently from the definition of $R^*$. Also, combinations of substituents and/or variables are permissible only if such combinations result in stable compounds, *i.e.*, compounds that can be isolated, characterized and tested for biological activity.

An "aureothin derivative" disclosed herein is a compound that shares the scaffold with the naturally occuring aureothin (formula **A**) but is not identical to it. In other words, the naturally occuring aureothin according to formula **A** above is excluded. Additionally, aureonitril is also excluded.

A "pharmaceutically acceptable salt" of a compound disclosed herein preferably is an acid or base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, $HOOC-(CH_2)_n-COOH$ where n is any integer from 0 to 4, *i.e.*, 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention, as are prodrugs of the compounds of formula (I) provided herein.

A "prodrug" is a compound that may not fully satisfy the structural requirements of the compounds provided herein, but is modified *in vivo,* following administration to a subject or patient, to produce a compound of formula (I) provided herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved

*in vivo* to generate the parent compounds.

A "substituent," as used herein, refers to a molecular moiety that is covalently bonded to an atom within a molecule of interest, e.g. to a compound of formula (I), (II), (III) or (IV) or a prodrug thereof. For example, a "ring substituent" may be a moiety such as a halogen, alkyl group, haloalkyl group or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, *i.e.*, a compound that can be isolated, characterized and tested for biological activity. When a substituent is oxo, *i.e.*, =O, then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

The expression alkyl preferably refers to a saturated, straight-chain or branched hydrocarbon group that contains from 1 to 20 carbon atoms, preferably from 1 to 12 carbon atoms, more preferably from 1 to 6 carbon atoms, for example a methyl, ethyl, propyl, isopropyl, n-butyl, iso-butyl, sec-butyl, *tert*-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, more preferably from 2 to 6 carbon atoms, for example an ethenyl, allyl, acetylenyl, propargyl, isoprenyl or hex-2-enyl group. Preferably, alkenyl groups have one or two, more preferably one, double bond(s) and alkynyl groups have one or two, more preferably one, triple bond(s).

Furthermore, the terms alkyl, alkenyl and alkynyl refer to groups in which one or more hydrogen atoms have been replaced each independently of the others by a halogen atom, preferably F or Cl, such as, for example, a 2,2,2-trichloroethyl or a trifluoromethyl group.

The expression heteroalkyl preferably refers to an alkyl, alkenyl or alkynyl group, for example heteroalkenyl, heteroalkynyl, in which one or more, preferably 1, 2 or 3 carbon atoms have been replaced each independently of the others by an oxygen, nitrogen, phosphorus, boron, selenium, silicon or sulphur atom, preferably oxygen, sulphur or nitrogen. The expression heteroalkyl, for example, refers to an alkoxy group. Furthermore, heteroalkyl preferably refers to a carboxylic acid or to a group derived from a carboxylic acid such as, for example, acyl, acylalkyl, alkoxycarbonyl, acyloxy, acyloxyalkyl, carboxyalkylamide, alkylcarbamoylalkyl, alkylcarbamoyloxyalkyl, alkylureidoalkyl, or alkoxycarbonyloxy.

Examples of heteroalkyl groups are groups of formulas -S-$Y^a$-L, -S-$Y^a$-CO-$NR^aR^b$, -$Y^a$-$NR^c$-CO-$NR^aR^b$, -$Y^a$-$NR^c$-CO-O-$R^d$, -$Y^a$-$NR^c$-CO-$R^d$, -$Y^a$-$NR^c$-CO-$NR^d$-L, -$Y^a$-$NR^c$-CS-$NR^d$-L, -$Y^a$-O-CO-$NR^aR^b$, -$Y^a$-CO-$NR^aR^b$, -O-$Y^a$-CO-$NR^aR^b$, -$Y^a$-$NR^c$-CO-L, , -$Y^a$-O-CO-O-$R^c$, -$Y^a$-O-CO-$R^c$, -$Y^a$-O-$R^c$, -$Y^a$-CO-L, -$Y^a$-$NR^aR^b$, $R^c$-S-$Y^a$-, $R^a$-N($R^b$)-$Y^a$-, $R^c$-CO-$Y^a$-, $R^c$-O-CO-$Y^a$-, $R^c$-CO-O-$Y^a$-, $R^c$-CO-N($R^b$)-$Y^a$-, $R^a$-N($R^b$)-CO-$Y^a$-, $R^c$-SO-$Y^a$-, $R^c$-$SO_2$-$Y^a$-, -$Y^a$-$NR^c$-$SO_2$-$NR^aR^b$, -$Y^a$-$SO_2$-$NR^aR^b$, -$Y^a$-$NR^c$-$SO_2$-$R^d$, $R^a$-O-CO-N($R^b$)-$Y^a$-, $R^a$-N($R^b$)-C(=$NR^d$)-N($R^c$)-$Y^a$-, $R^c$-S-CO-$Y^a$-, $R^c$-CO-S-$Y^a$-, $R^c$-S-CO-N($R^b$)-$Y^a$-, $R^a$-N($R^b$)-CO-S-$Y^a$-, $R^c$-S-CO-O-$Y^a$-, $R^c$-O-CO-S-$Y^a$-, $R^c$-S-CO-S-$Y^a$-; wherein $R^a$ being a hydrogen atom, a $C_1$-$C_6$alkyl, a $C_2$-$C_6$alkenyl, a $C_2$-$C_6$alkynyl , or is joined to $R^b$ to form a 4-to 10-membered cycloalkyl or heterocycloalkyl; $R^b$ being a hydrogen atom, a $C_1$-$C_6$alkyl, a $C_2$-$C_6$alkenyl or a $C_2$-$C_6$alkynyl , or taken together with $R^a$ to form a 4- to 10-membered cycloalkyl or heterocycloalkyl; $R^c$ being a hydrogen atom, an optionally substituted $C_1$-$C_8$alkyl, an optionally substituted $C_2$-$C_8$alkenyl or an optionally substituted $C_2$-$C_8$alkynyl ; $R^d$ being a hydrogen atom, optionally substituted $C_1$-$C_8$alkyl, optionally substituted $C_2$-$C_8$alkenyl or optionally substituted $C_2$-$C_8$alkynyl; L being a cycloalkyl, heterocycloalkyl, alkylcycloalkyl, heteroalkylcycloalkyl, optionally substituted aryl, optionally substituted heteroaryl, aralkyl, or heteroaralkyl; and $Y^a$ being a bond, a $C_1$-$C_6$alkylene, a $C_2$-$C_6$alkenylene or a $C_2$-$C_6$alkynylene group; each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, isobutyrylaminomethyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups. An example of a heteroalkylene group is a group of formulas -$CH_2CH(OH)$- or - CONH-.

The expression cycloalkyl preferably refers to a saturated or partially unsaturated cyclic group that contains one or more rings, preferably 1 or 2, containing from 3 to 14 ring carbon atoms, preferably from 3 to 10, more preferably 3, 4, 5, 6 or 7, ring carbon atoms. In an embodiment a partially unsaturated cyclic group has one, two or more double bonds, such as a cycloalkenyl group. The expression cycloalkyl preferably refers furthermore to groups in which one or more hydrogen atoms have been replaced each independently of the others by fluorine, chlorine, bromine or iodine atoms or by OH, =O, SH, =S, $NH_2$, =NH, CN or $NO_2$ groups, thus, for example, cyclic ketones such as, for example, cyclohexanone, 2-cyclohexenone or cyclopentanone. Further specific examples of a cycloalkyl group is a cyclopropyl,

cyclobutyl, cyclopentyl, spiro[4,5]decanyl, norbornyl, cyclohexyl, cyclopentenyl, cyclohexadienyl, decalinyl, bicyclo[4.3.0] nonyl, tetralin, cyclopentylcyclohexyl, fluorocyclohexyl or cyclohex-2-enyl group.

The expression heterocycloalkyl preferably refers to a cycloalkyl group as defined above in which one or more, preferably 1, 2 or 3, ring carbon atoms have been replaced each independently of the others by an oxygen, nitrogen, silicon, selenium, phosphorus or sulphur atom, preferably oxygen, sulphur or nitrogen. A heterocycloalkyl group has preferably 1 or 2 ring(s) containing from 3 to 10, more preferably 3, 4, 5, 6 or 7, ring atoms. The expression heterocycloalkyl preferably refers furthermore to groups in which one or more hydrogen atoms have been replaced each independently of the others by fluorine, chlorine, bromine or iodine atoms or by OH, =O, SH, =S, $NH_2$, =NH, CN or $NO_2$ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also a lactam, a lactone, a cyclic imide and a cyclic anhydride.

The expression alkylcycloalkyl preferably refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10, preferably 3, 4, 5, 6 or 7, carbon atoms, and one or two alkyl, alkenyl or alkynyl groups having 1 or 2 to 6 carbon atoms, the cyclic groups being optionally substituted.

The expression heteroalkylcycloalkyl preferably refers to alkylcycloalkyl groups as defined above in which one or more, preferably 1, 2 or 3, carbon atoms have been replaced each independently of the others by an oxygen, nitrogen, silicon, selenium, phosphorus or sulphur atom, preferably oxygen, sulphur or nitrogen. A heteroalkylcycloalkyl group preferably contains 1 or 2 ring systems having from 3 to 10, preferably 3, 4, 5, 6 or 7, ring atoms, and one or two alkyl, alkenyl, alkynyl or heteroalkyl groups having from 1 or 2 to 6 carbon atoms. Examples of such groups are alkylheterocycloalkyl, heterocycloalkylalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expression aryl or Ar preferably refers to an aromatic group that contains one or more rings containing from 6 to 14 ring carbon atoms, preferably from 6 to 10, more preferably 6, ring carbon atoms. The expression aryl (or Ar) preferably refers furthermore to groups in which one or more hydrogen atoms have been replaced each independently of the others by fluorine, chlorine, bromine or iodine atoms or by OH, SH, $NH_2$, CN or $NO_2$ groups. Examples are a phenyl, naphthyl, biphenyl, 2-fluorophenyl, anilinyl, 3-nitrophenyl or 4-hydroxyphenyl group.

The expression heteroaryl preferably refers to an aromatic group that contains one or more rings containing from 5 to 14 ring atoms, preferably from 5 to 10, more preferably 5 or 6, ring atoms, and contains one or more, preferably 1, 2, 3 or 4, oxygen, nitrogen, phosphorus or sulphur ring atoms, preferably O, S or N. Furthermore, the expression heteroaryl preferably refers to groups in which one or more hydrogen atoms have been replaced each independently of the others by fluorine, chlorine, bromine or iodine atoms or by OH, =O, SH, $NH_2$, =NH, CN or $NO_2$ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl preferably refers to a group containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1 H-indene, tetralin, dihydronaphthalene, indanone, phenylcyclopentyl, cumene, cyclohexylphenyl, fluorene and indan. An aralkyl group preferably contains one or two aromatic ring systems, 1 or 2 rings, containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl preferably refers to an aralkyl group as defined above in which one or more, preferably 1, 2, 3 or 4, carbon atoms have been replaced each independently of the others by an oxygen, nitrogen, silicon, selenium, phosphorus, boron or sulphur atom, preferably oxygen, sulphur or nitrogen, that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems, 1 or 2 rings, containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms.

Examples of heteroaralkyl groups are aryloxy, arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl, heteroalkylheteroarylalkyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expressions cycloalkyl, heterocycloalkyl, alkylcycloalkyl, heteroalkylcycloalkyl, aryl, heteroaryl, aralkyl and heteroaralkyl also refer to groups in which one or more hydrogen atoms of such groups have been replaced each independently of the others by fluorine, chlorine, bromine or iodine atoms or by OH, =O, SH, =S, $NH_2$, =NH, CN or $NO_2$ groups.

The expression "substituted" as used in conncetion with any group especially refers to a group in which one or more hydrogen atoms have been replaced each independently of the others by fluorine, chlorine, bromine or iodine atoms or by OH, =O, SH, =S, $NH_2$, =NH, CN or $NO_2$ groups. This expression refers furthermore to a group in which one or more hydrogen atoms have been replaced each independently of the others by an unsubstituted $C_1$-$C_6$alkyl, unsubstituted $C_2$-$C_6$alkenyl, unsubstituted $C_2$-$C_6$alkynyl, unsubstituted $C_1$-$C_6$heteroalkyl, unsubstituted $C_3$-$C_{10}$cycloalkyl, unsubstituted $C_2$-$C_9$heterocycloalkyl, unsubstituted $C_6$-$C_{10}$aryl, unsubstituted $C_1$-$C_9$heteroaryl, unsubstituted $C_7$-$C_{12}$aralkyl or unsubstituted $C_2$-$C_{11}$heteroaralkyl group.

The expression "halogen" or "halogen atom" as preferably used herein means fluorine, chlorine, bromine, iodine.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range.

The present invention preferably relates to an aureothin derivative according to formula (I) or (II):

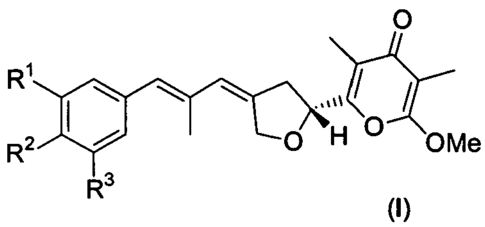

(I)

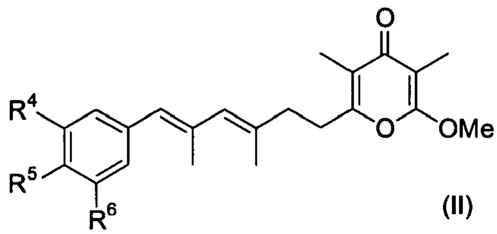

(II)

or a pharmacologically acceptable salt, solvate, hydrate or a pharmacologically acceptable formulation thereof, wherein

$R^1$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^1$ is taken together with $R^2$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^2$ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^2$ is taken together with $R^3$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^3$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes;

$R^4$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl,

an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^4$ is taken together with $R^5$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^5$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^5$ is taken together with $R^6$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^6$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes; provided that

(i) the naturally occuring aureothin according to formula A is excluded; and

(ii) $R^1$ and $R^3$ are not hydrogen if $R^2$ is cyano.

Preferred are aureothin derivatives - in the following also simply referred to as compounds - of formula (I) or (II), wherein $R^1$ and accordingly $R^4$ is a hydrogen atom.

Further preferred are compounds of formula (I) or (II), wherein $R^3$ and accordingly $R^6$ is a hydrogen atom.

Further preferred are compounds of formula (I), wherein $R^2$ is a hydrogen atom, halogen atom, methoxy, methyl, or trifluoromethyl.

Further preferred are compounds of formula (II), wherein $R^5$ is a hydrogen atom, halogen atom, cyano, nitro, methoxy, or methyl.

Further preferred are compounds of formula (I) or (II), wherein $R^1$ and $R^2$ and accordingly $R^4$ and $R^5$ are taken together to form a 6-membered carbocyclic ring.

Further preferred are compounds of formula (I) or (II), wherein $R^1$ and $R^2$ and accordingly $R^4$ and $R^5$ are taken together to form a 6-membered aromatic ring.

Further preferred are compounds of formula (I), wherein $R^1$ is a hydrogen atom;
$R^2$ is a hydrogen atom, halogen atom, methoxy, or methyl; and
$R^3$ is a hydrogen atom.

Further preferred are compounds of formula (II), wherein $R^4$ is a hydrogen atom;
$R^5$ is a hydrogen atom, halogen atom, cyano, methoxy, or methyl; and
$R^6$is a hydrogen atom.

Further preferred are compounds of formula (I) or (II), wherein $R^1$ and accordingly $R^4$ is a hydrogen atom;
$R^2$ and accordingly $R^5$ is a hydrogen atom; and
$R^3$ and accordingly $R^6$ is nitro.

It is to be noted that the present invention also encompasses all possible combinations of all preferred embodiments.

The compounds of formula (I) or (II) according to the present invention each have one or more improved properties, especially, improved antiproliferative, antibacterial, antifungal or cytostatic activity, low toxicity, low drug drug interaction, improved bioavailability, especially with regard to oral administration, improved metabolic stability, and improved solubility.

Aureothin derivatives provided herein exhibit high antitumor activity on cultured human tumor cell lines, i.e. an antiproliferative activity with an inhibition constant ($IC_{50}$) of preferably less than 1 $\mu$g/ml. Additionally, the aureothin

derivatives according to the present invention exhibit high antifungal activity and/or antimicrobial activity, i.e. an antifungal activity and/or antimicrobial activity with an inhibition constant ($IC_{50}$) of preferably less than than 1 $\mu$g/ml.

The activity and more specifically the pharmacological activity of the aureothin derivatives according to the present invention can be assessed using appropriate *in vitro* assays. For instance, the $IC_{50}$ values of the antitumor compounds according to the present invention may be determined via a depletion assay of cell growth. Preferred compounds of the invention have an $IC_{50}$ (half-maximal inhibitory concentration) of about 5 $\mu$g/ml or less, still more preferably an $IC_{50}$ of about 1 $\mu$g/ml or less, or even 0,5 $\mu$g/ml or less, even more preferably an $IC_{50}$ of about 0,1 $\mu$g/ml or less, or even 10 ng/ml or less in the assays mentioned above.

The therapeutic use of compounds of formula (I) or (II), their pharmacologically acceptable salts, prodrugs, solvates and hydrates and also formulations and pharmaceutical compositions containing the same are within the scope of the present invention. The present invention also relates to the use of those compounds of formula (I) or (II) as active ingredients in the preparation or manufacture of a medicament, especially, the use of compounds of formula (I) or (II), their pharmacologically acceptable salts, prodrugs or solvates and hydrates and also formulations and pharmaceutical compositions for the treatment of fungal infections or cancer as well as their use for the preparation of medicaments for the treatment of cancer or fungal infections.

The pharmaceutical compositions according to the present invention comprise at least one compound of formula (I) or (II) and, optionally, one or more carrier substances, excipients and/or adjuvants. Pharmaceutical compositions may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, antifungal, or anti-viral agent, an-anti histamine, a non-steroidal anti-inflammatory drug, a disease modifying anti-rheumatic drug, a cytostatic drug, a drug with smooth muscle activity modulatory activity or mixtures of the aforementioned.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g.,* intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate. Formulation for topical administration may be preferred for certain conditions such as, *e.g.,* in the treatment of skin conditions such as burns or itch.

Compositions intended for oral use may further comprise one or more components such as sweetening agents, flavoring agents, coloring agents and/or preserving agents in order to provide appealing and palatable preparations. Tablets contain the active ingredient in admixture with physiologically acceptable excipients that are suitable for the manufacture of tablets. Such excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, e.g., starch, gelatin or acacia, and lubricating agents such as, *e.g.,* magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules wherein the active ingredient is mixed with an inert solid diluent such as, *e.g.,* calcium carbonate, calcium phosphate or kaolin, or as soft gelatin capsules wherein the active ingredient is mixed with water or an oil medium such as,*e.g.*, peanut oil, liquid paraffin or olive oil.

Aqueous suspensions contain the active ingredient(s) in admixture with excipients suitable for the manufacture of aqueous suspensions. Such excipients include suspending agents suh as, *e.g*., sodium carboxymethylcellulose, methylcellulose, hydropropylmethylcellulose, sodium alginate, polyvinylpyrrolidone, gum tragacanth and gum acacia; and dispersing or wetting agents such as, *e.g*., naturally-occurring phosphatides such as lecithin, condensation products of an alkylene oxide with fatty acids such as polyoxyethylene stearate, condensation products of ethylene oxide with long chain aliphatic alcohols such as heptadecaethyleneoxycetanol, condensation products of ethylene oxide with partial esters derived from fatty acids and a hexitol such as polyoxyethylene sorbitol monooleate, or condensation products of ethylene oxide with partial esters derived from fatty acids and hexitol anhydrides such as polyethylene sorbitan monooleate. Aqueous suspensions may also comprise one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil such as, e.g.,

arachis oil, olive oil, sesame oil or coconut oil, or in a mineral oil such as liquid paraffin. The oily suspensions may contain a thickening agent such as beeswax, hard paraffin or cetyl alcohol. Sweetening agents such as those set forth above, and/or flavoring agents may be added to provide palatable oral preparations. Such suspensions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, such as sweetening, flavoring and coloring agents, may also be present.

Pharmaceutical compositions may also be in the form of oil-in-water emulsions. The oily phase may be a vegetable oil such as, *e.g.*, olive oil or arachis oil, a mineral oil such as, *e.g.*, liquid paraffin, or a mixture thereof. Suitable emulsifying agents include naturally-occurring gums such as, *e.g.*, gum acacia or gum tragacanth, naturally-occurring phosphatides such as, *e.g.*, soy bean lecithin, and esters or partial esters derived from fatty acids and hexitol, anhydrides such as, *e.g.*, sorbitan monoleate, and condensation products of partial esters derived from fatty acids and hexitol with ethylene oxide such as, *e.g.*, polyoxyethylene sorbitan monoleate. An emulsion may also comprise one or more sweetening and/or flavoring agents.

Syrups and elixirs may be formulated with sweetening agents, such as glycerol, propylene glycol, sorbitol or sucrose. Such formulations may also comprise one or more demulcents, preservatives, flavoring agents and/or coloring agents.

Compounds may be formulated for local or topical administration, such as for topical application to the skin or mucous membranes, such as in the eye. Formulations for topical administration typically comprise a topical vehicle combined with active agent(s), with or without additional optional components. Suitable topical vehicles and additional components are well known in the art, and it will be apparent that the choice of a vehicle will depend on the particular physical form and mode of delivery. Topical vehicles include water; organic solvents such as alcohols such as, *e.g.*, ethanol or isopropyl alcohol or glycerin; glycols such as, *e.g.*, butylene, isoprene or propylene glycol; aliphatic alcohols such as, *e.g.*, lanolin; mixtures of water and organic solvents and mixtures of organic solvents such as alcohol and glycerin; lipid-based materials such as fatty acids, acylglycerols including oils, such as, e.g., mineral oil, and fats of natural or synthetic origin, phosphoglycerides, sphingolipids and waxes; protein-based materials such as collagen and gelatin; silicone-based materials, both non-volatile and volatile; and hydrocarbon-based materials such as microsponges and polymer matrices. A composition may further include one or more components adapted to improve the stability or effectiveness of the applied formulation, such as stabilizing agents, suspending agents, emulsifying agents, viscosity adjusters, gelling agents, preservatives, antioxidants, skin penetration enhancers, moisturizers and sustained release materials. Examples of such components are described in Martindale--The Extra Pharmacopoeia (Pharmaceutical Press, London 1993) and Martin (ed.), Remington's Pharmaceutical Sciences. Formulations may comprise microcapsules, such as hydroxymethylcellulose or gelatin-microcapsules, liposomes, albumin microspheres, microemulsions, nanoparticles or nanocapsules.

A topical formulation may be prepared in a variety of physical forms including, for example, solids, pastes, creams, foams, lotions, gels, powders, aqueous liquids, emulsions, sprays and skin patches. The physical appearance and viscosity of such forms can be governed by the presence and amount of emulsifier(s) and viscosity adjuster(s) present in the formulation. Solids are generally firm and non-pourable and commonly are formulated as bars or sticks, or in particulate form; solids can be opaque or transparent, and optionally can contain solvents, emulsifiers, moisturizers, emollients, fragrances, dyes/colorants, preservatives and other active ingredients that increase or enhance the efficacy of the final product. Creams and lotions are often similar to one another, differing mainly in their viscosity; both lotions and creams may be opaque, translucent or clear and often contain emulsifiers, solvents, and viscosity adjusting agents, as well as moisturizers, emollients, fragrances, dyes/colorants, preservatives and other active ingredients that increase or enhance the efficacy of the final product. Gels can be prepared with a range of viscosities, from thick or high viscosity to thin or low viscosity. These formulations, like those of lotions and creams, may also contain solvents, emulsifiers, moisturizers, emollients, fragrances, dyes/colorants, preservatives and other active ingredients that increase or enhance the efficacy of the final product. Liquids are thinner than creams, lotions, or gels and often do not contain emulsifiers. Liquid topical products often contain solvents, emulsifiers, moisturizers, emollients, fragrances, dyes/colorants, preservatives and other active ingredients that increase or enhance the efficacy of the final product.

Suitable emulsifiers for use in topical formulations include, but are not limited to, ionic emulsifiers, cetearyl alcohol, non-ionic emulsifiers like polyoxyethylene oleyl ether, PEG-40 stearate, ceteareth-12, ceteareth-20, ceteareth-30, ceteareth alcohol, PEG-100 stearate and glyceryl stearate. Suitable viscosity adjusting agents include, but are not limited to, protective colloids or non-ionic gums such as hydroxyethylcellulose, xanthan gum, magnesium aluminum silicate, silica, microcrystalline wax, beeswax, paraffin, and cetyl palmitate. A gel composition may be formed by the addition of a gelling agent such as chitosan, methyl cellulose, ethyl cellulose, polyvinyl alcohol, polyquaterniums, hydroxyethylcellulose, hydroxypropylcellulose, hydroxypropylmethylcellulose, carbomer or ammoniated glycyrrhizinate. Suitable surfactants include, but are not limited to, nonionic, amphoteric, ionic and anionic surfactants. For example,

one or more of dimethicone copolyol, polysorbate 20, polysorbate 40, polysorbate 60, polysorbate 80, lauramide DEA, cocamide DEA, and cocamide MEA, oleyl betaine, cocamidopropyl phosphatidyl PG-dimonium chloride, and ammonium laureth sulfate may be used within topical formulations.

Suitable preservatives include, but are not limited to, antimicrobials such as methylparaben, propylparaben, sorbic acid, benzoic acid, and formaldehyde, as well as physical stabilizers and antioxidants such as vitamin E, sodium ascorbate/ascorbic acid and propyl gallate. Suitable moisturizers include, but are not limited to, lactic acid and other hydroxy acids and their salts, glycerin, propylene glycol, and butylene glycol. Suitable emollients include lanolin alcohol, lanolin, lanolin derivatives, cholesterol, petrolatum, isostearyl neopentanoate and mineral oils. Suitable fragrances and colors include, but are not limited to, FD&C Red No. 40 and FD&C Yellow No. 5. Other suitable additional ingredients that may be included in a topical formulation include, but are not limited to, abrasives, absorbents, anti-caking agents, anti-foaming agents, anti-static agents, astringents such as, e.g., witch hazel, alcohol and herbal extracts such as chamomile extract, binders/excipients, buffering agents, chelating agents, film forming agents, conditioning agents, propellants, opacifying agents, pH adjusters and protectants.

An example of a suitable topical vehicle for formulation of a gel is: hydroxypropylcellulose (2.1 %); 70/30 isopropyl alcohol/water (90.9%); propylene glycol (5.1 %); and Polysorbate 80 (1.9%). An example of a suitable topical vehicle for formulation as a foam is: cetyl alcohol (1.1 %); stearyl alcohol (0.5%); Quaternium 52 (1.0%); propylene glycol (2.0%); Ethanol 95 PGF3 (61.05%); deionized water (30.05%); P75 hydrocarbon propellant (4.30%). All percents are by weight.

Typical modes of delivery for topical compositions include application using the fingers; application using a physical applicator such as a cloth, tissue, swab, stick or brush; spraying including mist, aerosol or foam spraying; dropper application; sprinkling; soaking; and rinsing. Controlled release vehicles can also be used, and compositions may be formulated for transdermal administration as a transdermal patch.

A pharmaceutical composition may be formulated as inhaled formulations, including sprays, mists, or aerosols. Such formulations are particularly useful for the treatment of asthma or other respiratory conditions. For inhalation formulations, the compounds provided herein may be delivered via any inhalation methods known to those skilled in the art. Such inhalation methods and devices include, but are not limited to, metered dose inhalers with propellants such as CFC or HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent, *e.g.*, isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient.

Formulations or compositions suitable for nasal administration, wherein the carrier is a solid, include a coarse powder having a particle size, for example, in the range of 20 to 500 microns which is administered in the manner in which snuff is administered, *i.e.*, by rapid inhalation through the nasal passage from a container of the powder held close up to the nose. Suitable powder compositions include, by way of illustration, powdered preparations of the active ingredient thoroughly intermixed with lactose or other inert powders acceptable for intrabronchial administration. The powder compositions can be administered via an aerosol dispenser or encased in a breakable capsule which may be inserted by the patient into a device that punctures the capsule and blows the powder out in a steady stream suitable for inhalation.

Pharmaceutical compositions may also be prepared in the form of suppositories such as e.g., for rectal administration. Such compositions can be prepared by mixing the drug with a suitable non-irritating excipient that is solid at ordinary temperatures but liquid at the rectal temperature and will therefore melt in the rectum to release the drug. Suitable excipients include, for example, cocoa butter and polyethylene glycols.

Pharmaceutical compositions may be formulated as sustained release formulations such as, *i.e.,* a formulation such as a capsule that creates a slow release of modulator following administration. Such formulations may generally be prepared using well known technology and administered by, for example, oral, rectal or subcutaneous implantation, or by implantation at the desired target site. Carriers for use within such formulations are biocompatible, and may also be biodegradable; preferably the formulation provides a relatively constant level of modulator release. The amount of modulator contained within a sustained release formulation depends upon, for example, the site of implantation, the rate and expected duration of release and the nature of the condition to be treated or prevented.

For the treatment of microbial or fungal infections as well as for the treatment of cancer, the dose of the biologically active compound according to the invention may vary within wide limits and may be adjusted to individual

requirements. Active compounds according to the present invention are generally administered in a therapeutically effective amount. Preferred doses range from about 0.1 mg to about 140 mg per kilogram of body weight per day, about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination, *i.e.* other drugs being used to treat the patient, and the severity of the particular disease undergoing therapy.

Preferred compounds of the invention will have certain pharmacological properties. Such properties include, but are not limited to oral bioavailability, such that the preferred oral dosage forms discussed above can provide therapeutically effective levels of the compound *in vivo.*

Aureothin derivatives provided herein are preferably administered to a patient such as, *e.g.*, a human, orally or topically, and are present within at least one body fluid or tissue of the patient. Accordingly, the present invention further provides methods for treating patients suffering from cancer. As used herein, the term "treatment" encompasses both disease-modifying treatment and symptomatic treatment, either of which may be prophylactic, *i.e.*, before the onset of symptoms, in order to prevent, delay or reduce the severity of symptoms, or therapeutic, *i.e.*, after the onset of symptoms, in order to reduce the severity and/or duration of symptoms. Patients may include but are not limited to primates, especially humans, domesticated companion animals such as dogs, cats, horses, and livestock such as cattle, pigs, sheep, with dosages as described herein.

It is also within the present invention that the compounds according to the invention are used as or for the manufacture of a diagnostic agent, whereby such diagnostic agent is for the diagnosis of the diseases and conditions which can be addressed by the compounds of the present invention for therapeutic purposes as disclosed herein.

For various applications, the compounds of the invention can be labelled by isotopes, fluorescence or luminescence markers, antibodies or antibody fragments, any other affinity label like nanobodies, aptamers, peptides etc., enzymes or enzyme substrates. These labelled compounds of this invention are, for example, useful for mapping the location of tumor cells *in vivo, ex vivo, in vitro* and *in situ* such as, *e.g.* in tissue sections via autoradiography and as radiotracers for positron emission tomography (PET) imaging, single photon emission computerized tomography (SPECT) and the like to characterize those cells in living subjects or other materials. The labelled compounds according to the present invention may be used in therapy, diagnosis and other applications such as research tools *in vivo* and *in vitro,* in particular the applications disclosed herein.

Also the following methods for producing compounds of formula (I) or (II) (aureothin derivatives) lie within the scope of the present invention.

Compounds of formula (I) or (II) can be attained by mutasynthesis, or by chemically reacting aureothin derivatives.

The development of natural product based drugs is often hampered by their structural complexity. This fact precludes facile total synthetic access to analogues or the development of natural product libraries. Therefore, semisynthetic as well as biotechnological approaches are commonly pursued in pharmaceutical research and development (F. von Nussbaum, M. Brands, B. Hinzen, S. Weigand, D. Häbich, Angew. Chem. 2006, 118, 5194-5254; Angew. Chem. Int. Ed. 2006, 45, 5072-5129; I. Paterson, E. A. Anderson, Science 2005, 310, 451-453. c) D. J. Newman, G. M. Cragg, K. M. Snader, J. Nat. Prod. 2003, 66, 1022-1037). A very interesting strategy combines chemical semisynthesis with biosynthesis using genetically engineered microorganisms, a technique which occasionally has been termed mutational biosynthesis or in short mutasynthesis (Review: S. Weist, R. D. Süssmuth, Appl. Microbiol. Biotechnol. 2005, 68, 141-150) According to Rinehart (K. L. Rinehart, K. L. Pure Appl. Chem. 1977, 49, 1361-1384) mutasynthesis involves the generation of biosynthetic blocked mutants, feeding to and incorporation of a mutasynthon by the blocked mutants, isolation of the new metabolites which finally are evaluated for their biological activities.

For instance, compounds of formula (II) (deoxyaureothin (DOA) derivatives) can be produced by culturing a bacterium of the genus *Streptomyces thioletus* lacking the N-oxygenase gene *aurF* as well as the cytochrome P450 monooxygenase gene *aurH.* It is understood that the production of aureothine derivatives is not limited to the use of the particular organism described herein, which is given for illustrative purpose only. The invention also includes the use of any mutants which are capable of producing aureothin derivatives including natural mutants as well as artificial mutants, e.g. genetically manipulated mutants and the expression of the gene cluster responsible for biosynthesis in a producer strain or by heterologous expression in host strains, e.g. *S. lividans* ZX1 or *S. albus,* carrying plasmid pMZ01. Streptomyces albus pMZ01 has been deposited at DSMZ.

Aureothin derivatives can be obtained by mutasynthesis, e.g. by feeding the growing cultures of producer strain *S. albus* carrying plasmid pMZ01 with the respective mutasynthons, e.g. *para* or *meta* monosubstituted benzoic acid or SNAC derivatives instead of PNBA, e.g. mutasynthons selected from

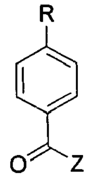

or

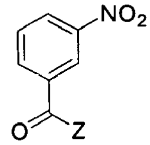

Z= OH or SNAC
R= Me, OMe, F, Cl,
Br, I, H, CN

SNAC=

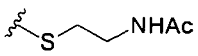

Similarly, 2-naphtoic acid or derivatives can be fed as mutasynthons to the growing cultures.

Aureothin derivatives according to formula (I) can, for example, be obtained by feeding the growing cultures of producer strain *S. lividans* ZX1 or *S. albus* carrying plasmid pHJ110, which producer strains express the aureothin cytochrome P450 monooxygenase AurH, described in J. He, M. Müller, C. Hertweck, J. Am. Chem. Soc. 2004, 126, 16742, with the deoxyaureothin derivatives according to formula (II). Both Streptomyces lividans ZX1/pHj110 and Streptomyces albus pHJ110 have been deposited at DSMZ.

Aureothins are produced in liquid culture, by growing the respective microorganism in media containing one or several different carbon sources, and one or different nitrogen sources. Also salts are essential for growth and production. Suitable carbon sources are different mono-, di-, and polysaccharides like glucose, sucrose, lactose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions.

Temperatures for growth and production are between 10°C to 40 °C, preferred temperatures are between 15°C and 37 °C, especially between 27°C to 33°C. The pH of the culture solution is from 4 to 8, preferably 6.5 and 7.5.

Aureothin derivatives having derivatized groups $R^1$, $R^2$, $R^3$, $R^4$, $R^5$ and/or $R^6$ can also be prepared using usual chemical reactions and synthesis methods known to a person skilled in the art.

For instance, a compound according to general formula (I) or (II) can be prepared by coupling Br-aureothin of formula (III) or Br-deoxyaureothin of formula (IV)

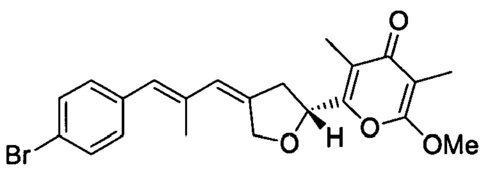

**(III)**

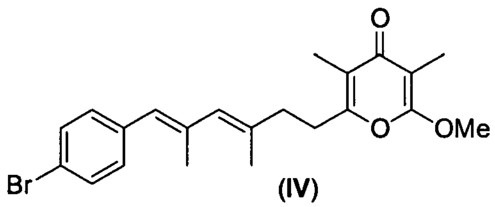

**(IV)**

with an organometal compound selected from the group consisting of $R^a$-$SnR^e_3$, $R^a$-$ZnR^e$, $R^a$-$AlR^e_2$, $R^a$-Cu, $R^a$-In, $MgR^e$, $BR^e_2$, $B(OR^f_2)$, $BF_3^-$, wherein $R^a$ is selected from alkyl, C2-C6 alkenyl, alkynyl, C2-C6 heteroalkyl, aryl, or aralkyl, especially aryl, C1-C6 alkyl, allyl, vinyl, C2-C6 alkinyl, $-(CH_2)_nOH$ (n= 1-3), $-(CH_2)_nCO_2H$ (n= 0-2), $-(CH_2)_nNH_2$ (n= 0-2), anticalins, antibodies, folic acid, fluorescence dyes, e.g. fluorescein or labels, or molecular probes, e.g. biotin; and each $R^e$ is independently selected from methyl, ethyl, butyl or cyclohexyl, and each $R^f$ is selected from hydrogen atom, pinacole or cresole. Similarly, I-aureothin or I-deoxyaureothin can be used.

Preferably, a Stille coupling is carried out, since Stille coupling in contrast to other catalyzed C-C coupling reactions like the Heck-Mizoroki and Suzuki-Miyaura reaction, proceeds under almost neutral conditions.

Concerning the derivatization with antibodies, see e.g. a) R. V. J. Chari, B. A. Martell, J. L. Gross, S. B. Cook, S. A. Shah, W. A. Blättler, S. J. McKenzie, V. S. Goldmacher, Cancer Res. 1992, 52, 127-131; b) K. Okamoto, K. Harada, S. Ikeyama, S. Iwasa, Jpn. J. Cancer Res. 1992, 83, 761-768; c) C. Liu, B. M. Tadayoni, L. A. Bourret, K. M. Mattocks, S. M. Derr, W. C. Widdison, N. L. Kedersha, P. D. Ariniello, V. S. Goldmacher, J. M. Lambert, W. A. Blättler, R. V. J.

Chari, Proc. Nat. Acad. Sci. USA 1996, 93, 8618-8623; and d) R. V. Madrigal, B. W. Zilkowski, C. R. Smith Jr., J. Chem. Ecol. 1985, 11, 501-506, and references cited therein.

As for the derivatization with anticalins, see e.g. a) S. Schlehuber, A. Skerra, Expert Opin Biol Ther. 2005, 5, 1453-1462 and b) S. Schlehuber, A. Skerra, Drug Discov Today. 2005, 10, 23-33.

With regard to folic acid, see e.g. a) A. R. Hilgenbrink, P. S. Low, J. Pharm. Sci.; 2005, 94, 2135-46 and b) C. P. Leamon, J. A. Reddy, Adv. Drug Deliv. Rev.; 2004, 56, 1127-1141.

For the attachment of linker groups, see e.g. P. Dubs, R. Stüssi, Helv. Chim. Acta 1976, 59, 1307-1311.

Further, a compound according to general formula (I) or (II) can be linked to tumor-associated ligands, e.g. anticalins, antibodies, folic acid, molecular probes or fluorescent dyes by acylation, sulfonation or phosphorylation. A more detailed discussion of the respective procedures can be found, e.g. in T. W. Greene, P. G. M. Wuts, Protective Groups in Organic Synthesis 3rd Edition, John Wiley & Sons, Inc., New York 1999, pp 149.

The present invention is now further illustrated by the following examples from which further features, embodiments and advantages of the present invention may be taken. However, these examples are by no means construed to be limiting to the present invention.

## General information

All reagents were purchased from commercial suppliers and used without further purification NMR spectra (in $CDCl_3$) were measured on Bruker Avance DRX 300 and DPX 500 instruments. Chemical shifts are reported in ppm. When peak multiplicities are reported, the following abbreviations are used: s, singlet; d, doublet; t, triplet; m, multiplet; br, broadened. The NMR numbering system refers to the general structure according to formula (**V**).

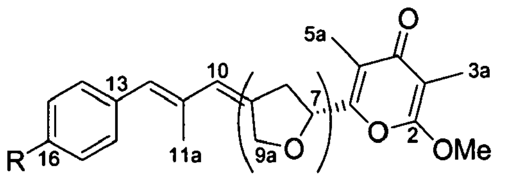

**(V)**

HR-MS (ESI) was recorded on a triple quadrupole mass spectrometer TSQ Quantum Ultra AM (Thermo Electron). LC-MS experiments were performed on a LCQ mass spectrometer equipped with an ESI source and an ion trap (Thermo Electron). Further analytical and preperative HPLC experiments were carried out on a Gilson machine. IR spectra were measured using a JASCO FT/IR-4100 spectrometer supplemented with the ATR system. Therefore, substances were charged as solids or thin films (highly concentrated solutions in volatile solvents). Optical rotation was determined with a JASCO P-1020 polarimeter supplemented with a thermostat.

Analytical thin layer chromatography (TLC) was carried out on Merck silica gel 60 F254 TLC plates. TLC visualization was accomplished using 254/366 nm UV light or charring solutions of molybdatophosphoric acid. Preparative column chromatography was performed on (0.04-0.063 mm, 230-400 mesh) silica gel normal phase.

**Example 1:** Producer strains

Construction of *aurF*/*aurH* null mutant

To generate a mutant that was suitable for the mutasynthesis of deoxyaureothin derivatives the *N*-oxygenase gene *aurF* as well as the cytochrome P450 monooxygenase gene *aurH* were deleted from the *aur* biosynthesis gene cluster. Both genes were subsequently excised from a cosmid bearing the entire *aur* gene cluster by PCR-targeting using specific primers as well as cloning strategies. Particularly, *aurH* was inactivated by partial digestion of a vector, pHJ65 containing a truncated *aur* gene cassette (*aurAGHB*), with *Nsp*I. Then, the inactivated *aurH* was cut with *Sgf*I and the yielded 2.845 kb fragment was ligated into the *Sgf*I site of the pHJ79 vector (pSET152 derivative; *aurF* null mutant; as described in J. He, C. Hertweck, J. Am. Chem. Soc. 2004, 126, 3694. The resulting plasmid, pMZ01, was introduced into S. *lividans* ZX1 and S. *albus* by PEG-induced protoplast transformation, and the resulting transformants were fermented in liquid production media. HPLC-MS monitoring of the extracts revealed that aureothin biosynthesis was fully abolished in both mutants (***Fig. 1***).

Complementation of *aurFlaurH* null mutant

The double knockout mutants were successfully complemented by coexpression of pMZ01 and pMZ04, an pWHM4* derivative constructed by cloning a PCR product of *aurF* into the *Xba*I site of pHJ110 (***Fig. 1***). In particular, the *aurF* gene including the native ribosome binding site (RBS) was amplified by PCR using primer FW (5' aatctagaAT-GCCACGACACCGCGGG 3'; *Xba*I restriction site) and RV (5' aatctagaACGCGGCGTCGGGGTCAACG 3'; *Xba*I restriction site). The PCR product was cloned into pGEM-T Easy Vector (Promega) for sequencing. The 1.16 kb *Xba*I fragment was then ligated into the *Xba*I site of pHJ110 (*aurH;* pWHM4* derivative; as described in J. He, M. Müller, C. Hertweck, J. Am. Chem. Soc. 2004, 126, 16742. The resulting construct, pMZ04, was cotransformed into *S. albus*::pMZ01 by PEG-induced protoplast transformation.

Production of deoxyaureothin

Furthermore, the mutants were supplemented with PNBA. The *S. albus* transformant proved to be the superior producer strain, yielding deoxyaureothin at c = 44.8 mg $L^{-1}$ within a 50 mL scale (***Fig. 1***).

**Example 2:** Analytical mutasynthesis

In order to successfully replace the nitro substituent in the naturally occuring aureothin we chose the concept of bioisosterism that is well known in drug design (C. J. Thompson, J. M. Ward and D. A. Hoopwood, Nature 1980, 286, 525). Bioisosters are substituents or groups that possess similar chemical and physical properties and may contribute to similar bioactivity profiles. These isosteric modifications have proven a successful method to vary the lead structure (K. Ylihonko and P. Mántsála, Microbiol. 1994, 140, 1359-1365).

We applied various mutasynthons ranging from para monosubstituted benzoic acids (e.g. terephthalic acid; terephthalic acid monomethylester) to *meta* substituted benzoic acids (e.g. m-nitro-BA), unsubstituted benzoic acid , and 2-naphthoic acid.

Analytical fermentation approaches for S. *albus*::pMZ01

50 mL of medium (R2YE or E1) supplemented with apramycin sulphate (c = 30 μg $mL^{-1}$) for plasmid selection was inoculated with 50 μL spore suspension (S. *albus*::pMZ01) and incubated at 30 °C for 20-24 hours with shaking (180-200 rpm). An inoculum (2.5 mL) of the preculture was transfered into 50 mL fresh medium and continuously incubated at 30 °C. After 2 days of growing the culture was fed with a precursor dissolved in 25% aq DMSO under pulse feeding on 2 following days (6 μmol / 3 μmol, c = 1 mg 100 $\mu L^{-1}$, saponification with 1 M NaOH) and incubated for another 5 days on a rotary shaker.

Mycelia and culture filtrate were extracted three times with an equal volume of EtOAc (80 mL). The combined organic layers were dried over $Na_2SO_4$ and concentrated to dryness under reduced pressure. The residue was dissolved in 1 mL MeOH and characterized via LC (Phenomenex Luna C18(2) 10 μm, 250 x 4.6 mm; eluent ACN/$H_2O$ gradient, flow rate 1 mL $min^{-1}$).

R2YE (C. J. Thompson, J. M. Ward and D. A. Hoopwood, Nature 1980, 286, 525): 103 g sucrose, 0,25 g $K_2SO_4$, 10.12 g $MgCl_2(6H_2O)$, 10 g glucose, 0.1 g casaminosacids, 5 g yeast extract, 5.73 g TES-buffer, 2 mL trace element solution, 1000 mL purified water

Trace element solution: 40 mg $ZnCl_2$, 200 mg $FeCl_3(6H_2O)$, 10 mg $CuCl_2(2H_2O)$, 10 mg $MnCl_2(4H_2O)$, 10 mg $Na_2B_4O_7(10H_2O)$, 10 mg $(NH_4)_6Mo_7O_{24}(4H_2O)$, 1000 mL purified water

E1 (K. Ylihonko and P. Mántsála, Microbiol. 1994, 140, 1359-1365): 20 g glucose, 20 g soluble starch, 5 g Pharmamedia®, 2.5 g yeast extract, 3 g $CaCO_3$, 1 g NaCl, 1 g $MgSO_4(7H_2O)$, 1 g $K_2HPO_4(3H_2O)$, 1000 mL purified water

Metabolite production was monitored by HPLC-MS.

Test of starter unit activation

Initially, we tested the impact of chemical starter unit activation. Therefore, N-acetyl cysteamine (NAC) adducts were synthesized as CoA mimics (J. Staunton, A. C. Sutkowski, J. Chem. Soc., Chem. Commun. 1991, 1110; T. Leaf, L. Cadapan, C. Carreras, R. Regentin, S. Ou, E. Woo, G. Ashley, P. Licari, Biotechnol. Prog. 2000, 16, 553) that readily diffuse into bacterial cells. However, when applying mutasynthons it proved to be irrelevant whether the analogue was supplied as SNAC thioester or as free acids. Apparently, the endogenous acyl-CoA ligase AurE is highly promiscuous. HPLC and HRMS data indicated the successful incorporation of the mutasynthon. The expected masses and isotope patterns were recorded for deoxyaureothin derivatives resulting from incorporation of all p-halogenated BAs, and the pseudohalide p-cyano-BA. Additionally, a clear incorporation could be observed for p-methyl-BA, p-methoxy-BA, the

unsubstituted benzoic acid, and 2-naphthoic acid.

**Example 3:** Large-scale mutasynthesis

Out of the various novel deoxyaureothin derivatives the most intruguing compounds from a chemical as well as a SAR dependant point of view and were chosen for upscaling approaches.

Large scale fermentations were performed in the case of the p-cyano-, p-halogen- and the naphthoate-substituted derivatives to obtain sufficient amounts for structure elucidation and biological testings.

Fermentations were performed using 2 x 20 L (per 30 L steel fermentor) or 60 L media (75 L steel fermentor) depending on the secondary metabolite. For production S. *albus*::pMZ01 was grown with stirring at 28 °C in R2YE or E1 media supplemented with 30 mg $L^{-1}$ apramycin sulfate for plasmid selection. A 3 day old seed culture of S. *albus*:: pMZ01 (1/100) was used to inoculate the fermentation media and the approach was incubated for 2 days. Then, the culture was fed with the precursor dissolved in 25% aq DMSO under pulse feeding on 2 following days (0.12 mmol $L^{-1}$ and 0.06 mmol $L^{-1}$, c = 1 mg 100 $\mu L^{-1}$, saponification with 1 M NaOH). After incubation for another 5 days the culture was neutralized with 1 N HCl and separated in mycelia und culture filtrate. After extraction with EtOAc the organic layers were dried with $Na_2SO_4$ and concentrated to dryness under reduced pressure.

The crude extracts were subjected to chromatography on silica gel using a $CHCl_3$/MeOH gradient as eluent. All fractions containing the preferred product were combined and further purified by preparative RP-HPLC (Phenomenex Luna C18(2) 10 $\mu$m, 250 x 21.2 mm; eluent: ACN/$H_2O$ gradient, flow rate 20 mL $min^{-1}$).

The p-cyano- and naphthoate-substituted derivatives were obtained from two fermentations in 20 L R2YE media. For the first one a production rate of 2 mg $L^{-1}$ and for the latter one of 1.8 mg $L^{-1}$ was observed. The p-halogenated derivatives harvested from fermentations in E1 media, which proved to be superior and cheaper, yielded in R = F: 5.4 mg $L^{-1}$, R = Cl: 3.1 mg $L^{-1}$, R = Br: 7.8 mg $L^{-1}$, R = I: 18 mg $L^{-1}$. Therefore again an approach of twofold 20 L was chosen except from the p-iodo substituted one for which a 60 L approach was performed. After neutralization of the fermentation approach with 1 N HCl mycelia and culture filtrate were extracted with ethyl acetate and the crude extracts were subjected to silica gel (normal phase) column chromatography using a $CHCl_3$/MeOH gradient. Final purification was achieved by preparative reverse phase HPLC and a ACH/$H_2O$ gradient as eluent.

Analytical data of novel metabolites

<u>deoxyaureonitrile</u> (light yellow solid)

$^1$H-NMR (300 MHz, $CDCl_3$): $\delta$ = 1.82 (s, 3H, 3a-$CH_3$), 1.88 (d, J(H,H) = 1.22 Hz, 3H, 9a-$CH_3$), 1.91 (d, J(H,H) = 1.18 Hz, 3H, 11a-$CH_3$), 1.94 (s, 3H, 5a-$CH_3$), 2.4 (br t, J(H,H) = 7.53 Hz, 2H, 8-$CH_2$), 2.75 (br t, J(H,H) = 7.57 Hz, 2H, 7-$CH_2$), 3.93 (s, 3H, OMe), 5.75 (br s, 1 H, 10-CH), 6.24 (br s, 1H, 12-CH), 7.31 (d, J(H,H) = 8.29 Hz, 2H, 14,18-CH), 7.57 (d, J(H,H) = 8.36 Hz, no determination of long range coupling constants, 2H, 15,17-CH) ppm; $^{13}$C-NMR (75 MHz, $CDCl_3$): $\delta$ = 6.82 (1 C, 3a-$CH_3$), 9.92 (1 C, 5a-$CH_3$), 18.03 (1 C, 9a-$CH_3$), 19.03 (1 C, 11 a-$CH_3$), 29.57 (1 C, 7-$CH_2$), 37.72 (1 C, 8-$CH_2$), 55.28 (1 C, OMe), 99.49 (1 C, 3-C), 109.56 (1 C, 16-C-CN), 118.58 (1C, 5-C), 119.03 (1 C, 13-C), 127.56 (1 C, 12-CH), 129.4 (2C, 14,18-CH), 130.36 (1C, 10-CH), 131.86 (2C, 15,17-CH), 136.37 (1C, 9-C), 138.47 (1C, 11-C), 142.59 (1 C, CN), 157.42 (1C, 6-C), 162.05 (1 C, 2-C), 180.83 (1 C, 4-C=O) ppm; IR: $\nu$ = 3044 (vw, =C-H), 3028 (w, =C-H), 2960/2931/2914/2878/2854/2833 (m-w, $CH_3$, $CH_2$), 2221 (s-m, C≡N), 1668 (s, C=O), 1616 (w, C=C), 1584 (vs, C=C), 1502 (w, C=C), 1461/1453/1415/1385/1373/1369 (m, $CH_3$, $CH_2$), 1337/1320/1250/1206/1164/1140/1043/1030/981 (m-w, C-O-C), 906 (m, =C-H), 855/833/799 (w, =C-H), 768 (m-w, =C-H), 704/665 (w, =C-H) $cm^{-1}$; HR-MS (ESI): calcd. for $C_{23}H_{26}N_1O_3$: 364.1907, observed: 364.1845.

<u>p-fluoro-DOA</u> (orange-yellow viscous oil)

$^1$H-NMR (300 MHz, $CDCl_3$): $\delta$ = 1.83 (s, 3H, 3a-$CH_3$), 1.88 (d, J(H,H) = 1.14 Hz, 6H, 9a-$CH_3$, 11a-$CH_3$), 1.95 (s, 3H, 5a-$CH_3$), 2.38 (br t, *J*(H,H) = 7.54 Hz, 2H, 8-$CH_2$), 2.74 (br t, *J*(H,H) = 7.59 Hz, 2H, 7-$CH_2$), 3.94 (s, 3H, OMe), 5.74 (br s, 1 H, 10-CH), 6.21 (br s, 1 H, 12-CH), 6.99 (m, no determination of long range or H-F coupling constants, 2H, 15,17-CH), 7.2 (m, no determination of long range or H-F coupling constants, 2H, 14,18-CH) ppm; $^{13}$C-NMR (75 MHz, $CDCl_3$): $\delta$ = 6.84 (1 C, 3a-$CH_3$), 9.92 (1 C, 5a-$CH_3$), 17.92 (1 C, 9a-$CH_3$), 18.71 (1C, 11a-$CH_3$), 29.69 (1C, 7-$CH_2$), 37.76 (1C, 8-$CH_2$), 55.28 (1C, OMe), 99.49 (1C, 3-C), 114.96 (d, 2C, *J*(C,F) = 21.28 Hz, 15,17-CH), 118.54 (1C, 5-C), 128.1 (1C, 12-CH), 130.44 (d, 2C, *J*(C,F) = 7.85 Hz, 14,18-CH), 130.73 (1C, 10-CH), 133.94 (d, 1C, *J*(C,F) = 3.39 Hz, 13-C), 134.88 (1C, 9-C), 134.94 (d, 1C, *J*(C,F) = 1.36 Hz, 11-C), 157.65 (1C, 6-C), 161.32 (d, 1C, *J*(C,F) = 259.01 Hz, 16-C-F), 162.09 (1C, 2-C), 180.92 (1C, 4-C=O) ppm; IR: $\nu$ = 3033 (vw, =C-H), 2956/2925/2857 (m-w, $CH_3$, $CH_2$), 1740 (br w, =C-H), 1666 (vs, C=O), 1594 (vs, C=C), 1539 (vw, C=C), 1505 (s, C=C), 1460/1409/1376 (m, $CH_3$, $CH_2$), 1227 (m, C-F), 1317/1246/1158/1094/1031 (br)/984 (m-w, C-O-C), 889/836(br) (w, =C-H), 768 (m, =C-H), 670 (w, =C-H) $cm^{-1}$;

HR-MS (ESI): calcd. for $C_{22}H_{26}FO_3$: 357.1860, observed: 357.1818.

p-chloro-DOA (orange-yellow viscous oil)

$^1$H-NMR (300 MHz, $CDCl_3$): $\delta$ = 1.83 (s, 3H, 3a-$CH_3$), 1.88 (t, J(H,H) = 1.63 Hz, 6H, 9a-$CH_3$, 11a-$CH_3$), 1.95 (s, 3H, 5a-$CH_3$), 2.39 (br t, *J*(H,H) = 7.54 Hz, 2H, 8-$CH_2$), 2.74 (br t, *J*(H,H) = 7.6 Hz, 2H, 7-$CH_2$), 3.94 (s, 3H, OMe), 5.74 (br s, 1H, 10-CH), 6.2 (br s, 1H, 12-CH), 7.16 (d, *J*(H,H) = 8.46 Hz, no determination of long range coupling constants, 2H, 14,18-CH), 7.26 (d, *J*(H,H) = 8.54 Hz, no determination of long range coupling constants, 2H, 15,17-CH) ppm; $^{13}$C-NMR (75 MHz, $CDCl_3$): $\delta$ = 6.8 (1 C, 3a-$CH_3$), 9.88 (1C, 5a-$CH_3$), 17.91 (1 C, 9a-$CH_3$), 18.77 (1 C, 11a-$CH_3$), 29.63 (1C, 7-$CH_2$), 37.72 (1 C, 8-$CH_2$), 55.24 (1 C, OMe), 99.47 (1 C, 3-C), 118.51 (1C, 5-C), 127.96 (1C, 12-CH), 128.2 (2C, 15,17-CH), 130.44 (2C, 14,18-CH), 130.63 (1 C, 10-CH), 131.94 (1 C, 16-C-Cl), 135.14 (1 C, 9-C), 135.72 (1 C, 11-C), 136.3 (1 C, 13-C), 157.56 (1C, 6-C), 162.04 (1C, 2-C), 180.87 (1C, 4-C=O) ppm; IR: $\nu$ = 3023 (vw, =C-H), 2950/2924/2854 (m-w, $CH_3$, $CH_2$), 1732 (br w, =C-H), 1666 (vs, C=O), 1596 (vs, C=C), 1539 (vw, C=C), 1489 (m, C=C), 1459/1408/1376 (m, $CH_3$, $CH_2$), 1341/1317/1246/1162/1136/1011/984 (m-w, C-O-C), 1091 (m, C-Cl), 889/852/801 (w, =C-H), 768 (m, =C-H), 723 (w, =C-H), 670 (br w, =C-H) $cm^{-1}$; HR-MS (ESI): calcd. for $C_{22}H_{26}ClO_3$: 373.1565, observed: 373.1520.

p-bromo-DOA (orange-yellow viscous oil)

$^1$H-NMR (300 MHz, $CDCl_3$): $\delta$ = 1.83 (s, 3H, 3a-$CH_3$), 1.87 (m, 6H, 9a-$CH_3$, 11a-$CH_3$), 1.94 (s, 3H, 5a-$CH_3$), 2.38 (br t, J(H,H) = 7.54 Hz, 2H, 8-$CH_2$), 2.74 (br t, J(H,H) = 7.59 Hz, 2H, 7-$CH_2$), 3.93 (s, 3H, OMe), 5.74 (br s, 1 H, 10-CH), 6.18 (br s, 1H, 12-CH), 7.1 (d, J(H,H) = 8.4 Hz, no determination of long range coupling constants, 2H, 14,18-CH), 7.42 (d, J(H,H) = 8.45 Hz, no determination of long range coupling constants, 2H, 15,17-CH) ppm; $^{13}$C-NMR (75 MHz, $CDCl_3$): $\delta$ = 6.84 (1 C, 3a-$CH_3$), 9.92 (1 C, 5a-$CH_3$), 17.96 (1 C, 9a-$CH_3$), 18.82 (1 C, 11a-$CH_3$), 29.66 (1C, 7-$CH_2$), 37.77 (1 C, 8-$CH_2$), 55.28 (1 C, OMe), 99.49 (1 C, 3-C), 118.54 (1 C, 5-C), 120.1 (1C, 16-C-Br), 128.03 (1C, 12-CH), 130.52 (2C, 14,18-CH), 130.67 (1C, 10-CH), 131.19 (2C, 15,17-CH), 135.24 (1 C, 9-C), 135.88 (1 C, 11-C), 136.8 (1 C, 13-C), 157.59 (1 C, 6-C), 162.07 (1C, 2-C), 180.89 (1C, 4-C=O) ppm; IR: $\nu$ = 3020 (vw, =C-H), 2950/2924/2854 (m-w, $CH_3$, $CH_2$), 1739 (br w, =C-H), 1666 (vs, C=O), 1595 (vs, C=C), 1539 (vw, C=C), 1485 (m-w, C=C), 1458/1408/1376 (m, $CH_3$, $CH_2$), 1341/1316/1246/1162/1136/1030(br)/1008/984 (m-w, C-O-C), 1072 (m-w, C-Br), 889/851/830/820/798 (w, =C-H), 768 (m, =C-H), 679 (br w, =C-H) $cm^{-1}$; HR-MS (ESI): calcd. for $C_{22}H_{26}BrO_3$: 417.1060, observed: 417.1025.

p-iodo-DOA (light yellow cristalline solid)

$^1$H-NMR (300 MHz, $CDCl_3$): $\delta$ = 1.83 (s, 3H, 3a-$CH_3$), 1.88 (m, 6H, 9a-$CH_3$, 11a-$CH_3$), 1.94 (s, 3H, 5a-$CH_3$), 2.38 (br t, 2H, *J*(H,H) = 7.55 Hz, 8-$CH_2$), 2.74 (br t, 2H, *J*(H,H) = 7.59 Hz, 7-$CH_2$), 3.93 (s, 3H, OMe), 5.74 (br s, 1 H, 10-CH), 6.16 (br s, 1H, 12-CH), 6.97 (d, *J*(H,H) = 8.28 Hz, no determination of long range coupling constants, 2H, 14,18-CH), 7.62 (d, J(H,H) = 8.38 Hz, no determination of long range coupling constants, 2H, 15,17-CH) ppm; $^{13}$C-NMR (75 MHz, $CDCl_3$): $\delta$ = 6.85 (1 C, 3a-$CH_3$), 9.93 (1 C, 5a-$CH_3$), 17.97 (1C, 9a-$CH_3$), 18.84 (1 C, 11 a-$CH_3$), 29.67 (1 C, 7-$CH_2$), 37.79 (1 C, 8-$CH_2$), 55.29 (1C, OMe), 91.49 (1 C, 16-C-I), 99.5 (1 C, 3-C), 118.55 (1C, 5-C), 128.13 (1C, 12-CH), 130.69 (1C, 10-CH), 130.79 (2C, 14,18-CH), 135.29 (1C, 9-C), 136.03 (1C, 11-C), 137.18 (2C, 15,17-CH), 137.39 (1C, 13-C), 157.58 (1C, 6-C), 162.07 (1C, 2-C), 180.89 (1C, 4-C=O) ppm; IR: $\nu$ = 3069/3055 (vw, =C-H), 3041/3020 (vw, =C-H), 2991/2953/2924/2854 (m-w, $CH_3$, $CH_2$), 1743 (br vw, =C-H), 1665 (vs, C=O), 1609 (vw, C=C), 1582 (vs, C=C), 1531 (vw, C=C), 1467/1415/1373 (s-m, $CH_3$, $CH_2$), 1320/1248/1164/1006/978 (m-w, C-O-C), 1065 (w, C-I), 909 (w, =C-H), 891 (m, =C-H), 851 (w, =C-H), 797/764 (m, =C-H), 723 (w, =C-H), 670 (br w, =C-H) $cm^{-1}$; HR-MS (ESI): calcd. for $C_{22}H_{26}IO_3$: 465.0921, observed: 465.0880.

2-naphthoate-DOA (shiny yellow viscous oil)

$^1$H-NMR (300 MHz, $CDCl_3$): $\delta$ = 1.85 (s, 3H, 3a-$CH_3$), 1.94 (d, *J*(H,H) = 1.28 Hz, 3H, 9a-$CH_3$), 1.97 (s, 3H, 5a-$CH_3$), 2.01 (d, *J*(H,H) = 1.2 Hz, 3H, 11a-$CH_3$), 2.41 (br t, *J*(H,H) = 7.56 Hz, 2H, 8-$CH_2$), 2.76 (br t, *J*(H,H) = 7.59 Hz, 2H, 7-$CH_2$), 3.95 (s, 3H, OMe), 5.82 (br s, 1H, 10-CH), 6.43 (br s, 1 H, 12-CH), 7.42 (m, 3H, Ar-H's), 7.69 (br s, 1 H, Ar-H), 7.79 (m, 3H, Ar-H's) ppm; $^{13}$C-NMR (75 MHz, $CDCl_3$)L $\delta$ = 6.83 (1 C, 3a-$CH_3$), 9.9 (1 C, 5a-$CH_3$), 17.98 (1 C, 9a-$CH_3$), 18.97 (1C, 11a-$CH_3$), 29.68 (1 C, 7-$CH_2$), 37.8 (1 C, 8-$CH_2$), 55.26 (1 C, OMe), 99.43 (1 C, 3-C), 118.48 (1 C, 5-C), 125.6/126.01/127.48/127.8 (4C, Ar-CH's), 129.27 (1 C, 12-CH), 130.93 (1 C, 10-CH), 131.99/133.31/135.42 (3C, Ar-C's), 134.94 (1 C, 9-C), 135.52 (1 C, 11-C), 157.66 (1C, 6-C), 162.07 (1C, 2-C), 180.89 (1C, 4-C=O) ppm; IR: $\nu$ = 3052 (w, =C-H), 2951/2925/2855 (m-w, $CH_3$, $CH_2$), 1666 (s, C=O), 1593 (vs, C=C), 1538 (vw, C=C), 1504 (vw, C=C), 1459/1409/1376 (m, $CH_3$, $CH_2$), 1341/1317/1246/1182/1163/1137/1031(br)/984 (m-w, C-O-C), 902 (w, =C-H), 860 (vw, =C-H), 818 (w, =C-H), 768/750 (m, =C-H), 667 (br vw, =C-H) $cm^{-1}$; HR-MS (ESI): calcd. for $C_{26}H_{29}O_3$: 389.2117, observed: 389.2123.

**Example 4:** *In vivo* biotransformation with AurH

Analytical scale transformation

The mutasynthesis of aureonitrile by administering *p*-cyano-BA to an *aurF* null mutant, *S. lividans*::pHJ79, as the only metabolite produced by this mutant albeit sampling different benzoates has been reported in M. Ziehl, J. He, H. M. Dahse, C. Hertweck, Angew. Chem. Int. Ed. Engl. 2005, 44, 1202. Nonetheless, we aimed at generating various aureothin derivatives that contain the chiral tetrahydrofuran moiety by pathway engineering. For this purpose, the aureothin cytochrome P450 monooxygenase AurH was heterologously expressed in *S. albus* and S. *lividans* using expression plasmid pHJ110, already described by He et al. in J. Am. Chem. Soc. 2004, 126, 16742. By supplementing a 50 mL host culture with deoxyaureothin dissolved in DMSO (1.3 μmol, c = 1 mg 25 $\mu L^{-1}$) the transformation into aureothin under the complete disappearing of starting material could be monitored by HPLC-MS (***Fig. 2***). Based on this result we started to use AurH as a target to introduce the furyl moiety into all of the metabolites generated by mutasynthesis.

50 mL of J medium supplemented with thiostreptone (c = 5 μg $mL^{-1}$) for plasmid selection was inoculated with 50 μL spore suspension (S. *albus*/pHJ110) and incubated at 30 °C for 2 days with shaking (180-200 rpm). An inoculum (2.5 mL) of the preculture was transferred into 50 mL fresh M10 medium and continuously incubated at 30 °C. After 2 days of growing the culture was fed with a precursor dissolved in DMSO (1.3 μmol, c = 1 mg 25 $\mu L^{-1}$) and incubated for another 5 days on a rotary shaker.

Mycelia and culture filtrate were extracted three times with an equal volume of EtOAc (80 mL). The combined organic layers were dried over $Na_2SO_4$ and concentrated to dryness under reduced pressure. The residue was dissolved in 1 mL MeOH and characterized via LC (Phenomenex Luna C18(2) 10 μm, 250 x 4.6 mm; eluent: ACN/$H_2O$ gradient, flow rate 1 mL $min^{-1}$).

Remarkably, oxygenase AurH showed a high tolerance towards artificial substrates and so the production of the appropriate aureothin analogues was detected by feeding deoxyaureonitrile, the p-halogen- as well as the naphthoate-substituted derivatives within the 50 mL scale.

Large scale transformation

For either a full structure elucidation and having enough material for investigations on SAR studies larger scale fermentations were performed in the case of the p-halogen- and naphthoate-substituted derivatives. Therefore, the metabolites were received from fermentations in 250 mL M10 media supplemented with the deoxyaureothin analogue dissolved in DMSO (6.5 μmol, c = 1 mg 25 $\mu L^{-1}$). Due to a less background of the host metabolism S. *albus*/pHJ110 was used as strain for *in vivo* biotransformation. In particular, fermentations were performed using 8 x 250 mL approaches. For production S. *albus*/pHJ110 was grown with stirring at 30 °C in J media supplemented with 5 μg $mL^{-1}$ thiostreptone for plasmid selection. After 2 days of growing an inoculum (12.5 mL) of the preculture was transferred into 250 mL fresh M10 media and the approach was incubated for 2 days under gently shaking (110 rpm). Then, the culture was fed with the precursor dissolved in DMSO (6.5 μmol, c = 1 mg 25 $\mu L^{-1}$). After incubation for another 5 days the culture was separated in mycelia und culture filtrate. After extraction with EtOAc the organic layers were dried with $Na_2SO_4$ and concentrated to dryness under reduced pressure.

The crude extracts were subjected to chromatography on silica gel using a $CHCl_3$/MeOH gradient as eluent. All fractions containing the preferred product were combined and further purified by semipreparative RP-HPLC (Macherey Nagel Nucleosil C18 100 5 μm, 250 x 10 mm; eluent: ACN/$H_2O$ gradient, flow rate 5 mL $min^{-1}$).

J (T. Kieser, M. J. Bibb, M. J. Buttner, K. F. Chater and D. A. Hopwood, Practical Streptomyces Genetics, John Innes Foundation, Norwich, 2000): 100 g sucrose, 10 g yeast extract, 30 g tryptone soya broth, 10 g $MgCl_2(6H_2O)$, 1000 ml purified water

M10 (T. Kieser, M. J. Bibb, M. J. Buttner, K. F. Chater and D. A. Hopwood, Practical Streptomyces Genetics, John Innes Foundation, Norwich, 2000): 4 g glucose, 4 g yeast extract, 10 g malt extract, 1000 ml purified water, pH = 7.3

Every 8 x 250 mL approaches were done, meanwhile for the p-iodo-derivative which was used to increase the feeding amount to 250 mL culture. Different amounts of the p-iododeoxyaureothin analogue (1. 6.5 μmol, 2. 13 μmol, 3. 26 μmol, 4. 52 μmol, 5. 104 μmol) yielded in a optimum of production for the lowest quantity of the suitable aureothin analogue (1. 74%, 2. 52 %, 3. 52%, 4. 40%, 5. 46%) quantified by HPLC.

Analytical data of novel metabolites

p-fluoro-aureothin (light yellow solid)

$[\alpha]_D^{20}$ = - 23.0 (2.24 mg $mL^{-1}$ in MeOH); $^1$H-NMR (500 MHz, $CDCl_3$): δ = 1.83 (s, 3H, 3a-$CH_3$), 1.96 (s, 3H,

11a-$CH_3$), 2.01 (s, 3H, 5a-$CH_3$), 2.89 (br dd, *J*(H,H) = 6.12/15.86 Hz, 1H, 8-$CH_AH_B$), 3.01 (br dd, *J*(H,H) = 7.14/15.9 Hz, 1H, 8-$CH_AH_B$), 3.92 (s, 3H, OMe), 4.71 (br d, *J*(H,H) = 13.93 Hz, 1 H, 9a-$CH_AH_B$), 4.83 (br d, *J*(H,H) = 13.93 Hz, 1H, 9a-$CH_AH_B$), 5.11 (t, *J*(H,H) = 6.86 Hz, 1 H, 7-CH), 6.13 (br s, 1 H, 10-CH), 6.27 (br s, 1 H, 12-CH), 7.01 (t, J(H,H) = 8.62, no determination of long range or H-F coupling constants, 2H, 15,17-CH), 7.19 (m, no determination of long range or H-F coupling constants, 2H, 14,18-CH) ppm; $^{13}$C-NMR (125 MHz, $CDCl_3$): $\underline{\delta}$ = 6.88 (1C, 3a-$CH_3$), 9.4 (1 C, 5a-$CH_3$), 17.36 (1C, 11a-$CH_3$), 38.21 (1C, 8-$CH_2$), 55.24 (1C, OMe), 70.1 (1C, 9a-$CH_2$), 73.25 (1C, 7-CH), 99.97 (1C, 3-C), 115.16 (d, 2C, *J*(C,F) = 21.5 Hz, 15,17-CH), 119.98 (1C, 5-C), 126.38 (1C, 10-CH), 129.46 (1C, 12-CH), 130.58 (d, 2C, *J*(C,F) = 7.92 Hz, 14,18-CH), 133.48 (d, 1C, *J*(C,F) = 3.4 Hz, 13-C), 134.73 (1C, 11-C), 138.18 (1C, 9-C), 155.02 (1C, 6-C), 161.55 (d, 1C, *J*(C,F) = 246.89 Hz, 16-C-F), 162.09 (1C, 2-C), 180.6 (1C, 4-C=O) ppm; IR: $\underline{\nu}$ = 2957/2920/2865 (w, $CH_3$, $CH_2$), 1748 (br w, =C-H), 1670 (s, C=O), 1608 (vs, C=C), 1539 (vw, C=C), 1505 (s-m, C=C), 1456/1413/1377/1369 (m-w, $CH_3$, $CH_2$), 1221 (m-w, C-F), 1321/1250/1181/1156/1047/1031/986/970 (m-w, C-O-C), 926 (vw, =C-H), 891/816/766/733 (w, =C-H), 665 (vw, =C-H) $cm^{-1}$; HR-MS (ESI): calcd. for $C_{22}H_{24}FO_4$: 371.1659, observed: 371.1643.

<u>p-chloro-aureothin</u> (light yellow solid)

$[\alpha]_D^{20}$ = + 25.7 (6.38 mg $mL^{-1}$ in MeOH); $^1$H-NMR (500 MHz, $CDCl_3$): $\underline{\delta}$ = 1.83 (s, 3H, 3a-$CH_3$), 1.96 (s, 3H, 11a-$CH_3$), 2.0 (s, 3H, 5a-$CH_3$), 2.89 (br dd, *J*(H,H) = 5.69/15.74 Hz, 1 H, 8-$CH_AH_B$), 3.01 (br dd, *J*(H,H) = 7.02/15.78 Hz, 1H, 8-$CH_AH_B$), 3.91 (s, 3H, OMe), 4.7 (br d, *J*(H,H) = 13.93 Hz, 1 H, 9a-$CH_AH_B$), 4.82 (br d, *J*(H,H) = 13.95 Hz, 1 H, 9a-$CH_AH_B$), 5.1 (t, *J*(H,H) = 6.82 Hz, 1H, 7-CH), 6.13 (br s, 1H, 10-CH), 6.25 (br s, 1H, 12-CH), 7.15 (d, J(H,H) = 8.23 Hz, 2H, 14,18-CH), 7.28 (d, *J*(H,H) = 8.27 Hz, 2H, 15,17-CH) ppm; $^{13}$C-NMR (125 MHz, $CDCl_3$): $\underline{\delta}$ 6.87 (1C, 3a-$CH_3$), 9.38 (1 C, 5a-$CH_3$), 17.43 (1 C, 11a-$CH_3$), 38.2 (1C, 8-$CH_2$), 55.23 (1C, OMe), 70.08 (1C, 9a-$CH_2$), 73.24 (1 C, 7-CH), 99.95 (1C, 3-C), 119.98 (1 C, 5-C), 126.31 (1C, 10-CH), 128.38 (2C, 15,17-CH), 129.3 (1C, 12-CH), 130.25 (2C, 14,18-CH), 132.46 (1C, 16-C-Cl), 135.45 (1 C, 11-C), 135.87 (1 C, 13-C), 138.58 (1 C, 9-C), 154.95 (1C, 6-C), 162.07 (1C, 2-C), 180.57 (1C, 4-C=O) ppm; IR: $\underline{\nu}$ = 2956/2920/2855 (m-w, $CH_3$, $CH_2$), 1736 (br vw, =C-H), 1668 (s, C=O), 1606 (vs, C=C), 1536 (vw, C=C), 1489 (m-w, C=C), 1457/1413/1368 (m, $CH_3$, $CH_2$), 1321/1252/1182/1157/1045/1030/1007/986/969 (s-m, C-O-C), 1090 (m, C-Cl), 925 (vw, =C-H), 897/805/766/733 (w, =C-H), 706 (vw, =C-H), 665 (w-vw, =C-H) $cm^{-1}$; HR-MS (ESI): calcd. for $C_{22}H_{24}ClO_4$: 387.1358, observed: 387.1352.

<u>p-bromo-aureothin</u> (light yellow solid)

$[\alpha]_D^{20}$ = + 27.4 (4.8 mg $mL^{-1}$ in MeOH); $^1$H-NMR (500 MHz, $CDCl_3$): $\underline{\delta}$ = 1.82 (s, 3H, 3a-$CH_3$), 1.95 (br s, 3H, 11a-$CH_3$), 2.0 (s, 3H, 5a-$CH_3$), 2.89 (br dd, *J*(H,H) = 6.15/15.93 Hz, 1H, 8-$CH_AH_B$), 3.01 (br dd, J(H,H) = 6.64/15.94 Hz, 1H, 8-$CH_AH_B$), 3.91 (s, 3H, OMe), 4.7 (br d, *J*(H,H) = 13.93 Hz, 1 H, 9a-$CH_AH_B$), 4.82 (br d, *J*(H,H) = 13.99 Hz, 1H, 9a-$CH_AH_B$), 5.1 (t, *J*(H,H) = 6.88 Hz, 1H, 7-CH), 6.13 (br s, 1H, 10-CH), 6.23 (br s, 1H, 12-CH), 7.09 (d, *J*(H,H) = 8.4 Hz, no determination of long range coupling constants, 2H, 14,18-CH), 7.44 (d, *J*(H,H) = 8.44 Hz, no determination of long range coupling constants, 2H, 15,17-CH) ppm; $^{13}$C-NMR (125 MHz, $CDCl_3$): $\underline{\delta}$ = 6.87 (1 C, 3a-$CH_3$), 9.39 (1 C, 5a-$CH_3$), 17.44 (1 C, 11a-$CH_3$), 38.21 (1C, 8-$CH_2$), 55.23 (1C, OMe), 70.09 (1C, 9a-$CH_2$), 73.24 (1C, 7-CH), 99.96 (1C, 3-C), 119.99 (1C, 5-C), 120.59 (1C, 16-C-Br), 126.32 (1C, 10-CH), 129.33 (1C, 12-CH), 130.57 (2C, 14.18-CH), 131.33 (2C, 15,17-CH), 135.55 (1C, 11-C), 136.32 (1C, 13-C), 138.64 (1C, 9-C), 154.93 (1C, 6-C), 162.07 (1C, 2-C), 180.58 (1C, 4-C=O) ppm; IR: $\underline{\nu}$ = 2954/2921/2856 (m-w, $CH_3$, $CH_2$), 1747 (br vw, =C-H), 1668 (s, C=O), 1605 (vs, C=C), 1536 (vw, C=C), 1486 (w, C=C), 1457/1412/1377/1368 (m, $CH_3$, $CH_2$), 1320/1251/1182/1158/1044/1032/1004/986/970 (s-m, C-O-C), 1073 (w, C-Br), 925 (vw, =C-H), 896/802/766/732 (w, =C-H), 665 (vw, =C-H) $cm^{-1}$; HR-MS (ESI): calcd. for $C_{22}H_{24}BrO_4$: 431.0852, observed: 431.0850.

<u>p-iodo-aureothin</u> (light yellow cristalline solid)

$[\alpha]_D^{20}$ = + 28.5 (5.17 mg $mL^{-1}$ in MeOH); $^1$H-NMR (300 MHz, $CDCl_3$): $\underline{\delta}$ = 1.83 (s, 3H, 3a-$CH_3$), 1.96 (d, J(H, H) = 1.13 Hz, 3H, 11a-$CH_3$), 2.0 (s, 3H, 5a-$CH_3$), 2.89 (br dd, *J*(H,H) = 6.17/6.48/16.12 Hz, 1 H, 8-$CH_AH_B$), 3.01 (br dd, J(H,H) = 7.31/15.95 Hz, 1H, 8-$CH_AH_B$), 3.91 (s, 3H, OMe), 4.69 (br d, J(H,H) = 14.0 Hz, 1 H, 9a-$CH_AH_B$), 4.82 (br d, J (H,H) = 14.08 Hz, 1 H, 9a-$CH_AH_B$), 5.1 (t, J(H,H) = 6.72 Hz, 1 H, 7-CH), 6.12 (br s, 1 H, 10-CH), 6.22 (br s, 1H, 12-CH),

6.96 (d, J(H,H) = 8.28 Hz, no determination of long range coupling constants, 2H, 14,18-CH), 7.64 (d, J(H,H) = 8.41 Hz, no determination of long range coupling constants, 2H, 15,17-CH) ppm; $^{13}$C-NMR (75 MHz, $CDCl_3$): δ = 6.88 (1 C, 3a-$CH_3$), 9.4 (1 C, 5a-$CH_3$), 17.46 (1 C, 11a-$CH_3$), 38.23 (1C, 8-$CH_2$), 55.25 (1 C, OMe), 70.1 (1C, 9a-$CH_2$), 73.26 (1 C, 7-CH), 92.03 (1 C, 16-C-I), 100.0 (1 C, 3-C), 120.01 (1 C, 5-C), 126.34 (1C, 10-CH), 129.44 (1 C, 12-CH), 130.81 (2C, 14,18-CH), 135.68 (1C, 11-C), 136.92 (1C, 13-C), 137.33 (2C, 15,17-CH), 138.7 (1 C, 9-C), 154.95 (1 C, 6-C), 162.09 (1C, 2-C), 180.59 (1 C, 4-C=O) ppm; IR: ν = 2950/2920/2859 (m-w, $CH_3$, $CH_2$), 1747 (br vw, =C-H), 1668 (s, C=O), 1605 (vs, C=C), 1536 (vw, C=C), 1482 (w, C=C), 1456/1411/1377/1367 (m, $CH_3$, $CH_2$), 1320/1251/1181/1158/1043/1033/1000/986 (s-m, C-O-C), 1063 (vw, C-I), 925 (vw, =C-H), 896/800/767/732 (w, =C-H), 665 (vw, =C-H) $cm^{-1}$; HR-MS (ESI): calcd. for $C_{22}H_{24}IO_4$: 479.0714, observed: 479.0694.

2-naphthoate-aureothin (light yellow solid)

$[\alpha]_D^{20} = +37.3$ (3.06 mg $mL^{-1}$ in MeOH); $^1$H-NMR (500 MHz, $CDCl_3$): δ = 1.84 (s, 3H, 3a-$CH_3$), 2.02 (s, 3H, 5a-$CH_3$), 2.08 (br s, 11a-$CH_3$), 2.92 (br dd, J(H,H) = 5.59/5.98/15.84 Hz, 1H, 8-$CH_AH_B$), 3.05 (br dd, J(H,H) = 6.54/15.87 Hz, 1 H, 8-$CH_AH_B$), 3.94 (s, 3H, OMe), 4.76 (br d, J(H,H) = 13.98 Hz, 1H, 9a-$CH_AH_B$), 4.88 (br d, J(H,H) = 13.96 Hz, 1 H, 9a-$CH_AH_B$), 5.13 (t, J(H,H) = 6.8 Hz, 1H, 7-CH), 6.21 (br s, 1 H, 10-CH), 6.48 (br s, 1 H, 12-CH), 7.36 (m, 1 H, Ar-CH), 7.44 (m, 2H, Ar-CH's), 7.68 (br s, 1H, Ar-CH), 7.79 (m, 3H, Ar-CH's) ppm; $^{13}$C-NMR (125 MHz, $CDCl_3$): δ = 6.89 (1C, 3a-$CH_3$), 9.41 (1 C, 5a-$CH_3$), 17.63 (1 C, 11a-$CH_3$), 38.27 (1C, 8-$CH_2$), 55.28 (1C, OMe), 70.18 (1C, 9a-$CH_2$), 73.24 (1C, 7-CH), 99.96 (1C, 3-C), 119.95 (1C, 5-C), 126.65 (1C, 10-CH), 125.93/126.23/127.33/127.6/127.7/-127.88 (7C, Ar-CH's), 130.69 (1C, 12-CH), 132.2/133.28 (2C, Ar-C's), 134.98 (1C, 13-C), 135.25 (1C, 11-C), 138.21 (1C, 9-C), 155.1 (1C, 6-C), 162.12 (1C, 2-C), 180.63 (1C, 4-C=O) ppm; IR: ν = 3052 (w, =C-H), 2957/2924/2855 (m-w, $CH_3$, $CH_2$), 1747 (w, =C-H), 1662 (s, C=O), 1584 (vs, C=C), 1537 (vw, C=C), 1505 (vw, C=C), 1463/1412/1374 (m, $CH_3$, $CH_2$), 1324/1257/1161/1049/1034/976 (s-m, C-O-C), 898 (w, =C-H), 862 (vw, =C-H), 816/767/749 (m-w, =C-H), 668 (vw, =C-H) $cm^{-1}$; HR-MS (ESI): calcd. for $C_{26}H_{26}O_4$: 403.1904, observed: 403.1891.

**Example 5:** Characterization of novel metabolites

The structures of the new compounds were fully elucidated by 1 D and 2D NMR experiments, mass spectrometry, and infrared spectroscopy. NMR shifts of the core structures were similar to that of aureothin (A) and deoxyaureothin (B), respectively. Conspicuous shifts leading to a different substitution pattern of the phenyl group are highlighted in ***Tables* 1 and 2.** The influence of the groups inducing different I- and M-effects could be clearly seen in shifts of the neighbouring atoms. Furthermore, the fluoro-substituted analogue showed typical C-F coupling constants up to 6 atoms in the $^{13}$C-NMR ranging between 259 Hz and 3 Hz. Two dimensional NMR- as well as NOESY-experiments revealed same connectivities compared to aureothin (A) and deoxyaureothin (B).

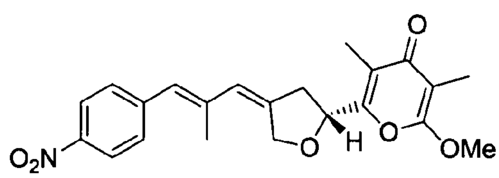

**(A)**

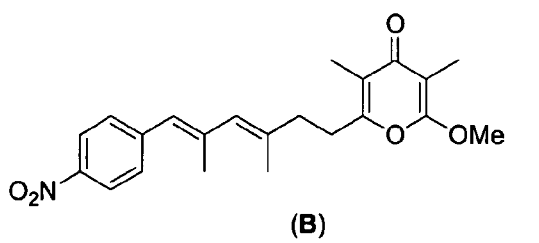

**(B)**

HR-MS measurements offered the adequate formular masses. In addition the chloro- and bromo-derivatives showed the typical isotopic pattern ("A+2"). By IR-spectroscopy the vibration of the nitrile group as well as the C-Hal vibrations could be clearly observed Further hints for the para-substitution of the phenyl head group could be found within the fingerprint (ca. 850-810 $cm^{-1}$).

**Table 1:** Diagnostic $^1$H--NMR shifts in ppm for deoxyaureothin and aureothin derivatives.

| | 14,18-CH | 15,17-CH |
|---|---|---|
| **deoxyaureothin analogues** | | |
| Ar-CN | 7.31 (d, *J*(H,H) = 8.29 Hz) | 7.57 (d, *J*(H,H) = 8.36 Hz) |
| Ar-F | 7.2 (m) | 6.99 (m) |
| Ar-Cl | 7.16 (d, *J*(H,H) = 8.46 Hz) | 7.26 (d, *J*(H,H) = 8.54 Hz) |
| Ar-Br | 7.1 (d, *J*(H,H) = 8.4 Hz) | 7.42 (d, *J*(H,H) = 8.45 Hz) |
| Ar-I | 6.97 (d, *J*(H,H) = 8.28 Hz) | 7.62 (d, *J*(H,H) = 8.38 Hz) |
| **aureothin analogues** | | |
| Ar-F | 7.1 (m) | 7.1 (m) |
| Ar-Cl | 7.16 (d, *J*(H,H) = 8.23 Hz) | 7.28 (d, *J*(H,H) = 8.27 Hz) |
| Ar-Br | 7.09 (d, *J*(H,H) = 8.4 Hz) | 7.44 (d, *J*(H,H) = 8.44 Hz) |
| Ar-I | 6.96 (d, *J*(H,H) = 8.28 Hz) | 7.64 (d, *J*(H,H) = 8.41 Hz) |

**Table 2:** Diagnostic $^{13}$C-NMR shifts in ppm for deoxyaureothin and aureothin derivatives.

| | 13-C | 14,18-C | 15,17-C | 16-C |
|---|---|---|---|---|
| **deoxyaureothin analogues** | | | | |
| Ar-CN | 119.03 | 129.4 | 131.86 | 109.56 |
| Ar-F | 133.94 (d, *J*(C,F) = 3.39 Hz) | 130.44 (d, *J*(C,F) = 7.85 Hz) | 114.96 (d, *J*(C,F) = 21.28 Hz) | 161.32 (d, *J*(C,F) = 259.01 Hz) |
| Ar-Cl | 136.3 | 130.44 | 128.2 | 131.94 |
| Ar-Br | 136.8 | 130.52 | 131.19 | 120.1 |
| Ar-I | 137.39 | 130.79 | 137.18 | 91.49 |
| **aureothin analogues** | | | | |
| Ar-F | 133.48 (d, *J*(C,F) = 3.4 Hz) | 130.58 (d, *J*(C,F) = 7.92 Hz) | 115.16 (d, *J*(C,F) = 21.5 Hz) | 161.55 (d, *J*(C,F) = 246.89 Hz) |
| Ar-Cl | 135.87 | 130.25 | 128.38 | 132.46 |
| Ar-Br | 136.32 | 130.57 | 131.33 | 120.59 |
| Ar-I | 136.92 | 130.81 | 137.33 | 92.03 |

**Example 6:** Bioassays

Cells and culture conditions

| *Cells* | *cell culture medium* |
|---|---|
| Huvec (ATCC CRL-1730) | DMEM (CAMBREX 12-614F) |
| K-562 (DSM ACC 10) | RPMI 1640 (CAMBREX 12-167F) |
| HeLa (DSM ACC 57) | RPMI 1640 (CAMBREX 12-167F) |

Cells were grown in the appropiate cell culture medium supplemented with 10 mL $L^{-1}$ ultraglutamine 1 (Cambrex 17-605E/U1), 500 μL $L^{-1}$ gentamicin sulfate (CAMBREX 17-518Z), and 10% heat inactivated fetal bovine serum (PAA A15-144) at 37°C in high density polyethylene flasks (NUNC 156340).

Antiproliferative assay

The test substances are dissolved in DMSO before being diluted in DMEM. Adherent K-562 cells were harvested

at the logarithmic growth phase after soft trypsinization, using 0.25% trypsin in PBS containing 0.02% EDTA (Biochrom KG L2163). For each experiment approximately 10,000 cells were seeded with 0.1 mL culture medium per well of the 96-well microplates (NUNC 167008).

Cytotoxic assay

For the cytotoxic assay HeLa cells were 48 hours preincubated without the test substances. The dilutions of the compounds were carried out carefully on the subconfluent monolayers of HeLa cells after the preincubation time.

Condition of incubation

The cells were incubated with dilutions of the test substances for 72 hours at 37°C in a humidified atmosphere and 5% $CO_2$.

Method of evaluation

For estimating the influence of chemical compounds on cell proliferation of K-562 we determinate the numbers of viable cells present in multiwell plates via CellTiter-Blue® assay. It uses the indicator dye resazurin to measure the metabolic capacity of cells as indicator of cell viability. Viable cells of untreated control retain the ability to reduce resazurin into resorufin, which is highly fluorescent. Nonviable cells rapidly lose metabolic capacity, do not reduce the indicator dye, and thus do not generate a fluorescent signal. Under our experimental conditions, the signal from the CellTiter-Blue® reagent is proportional to the number of viable cells.

The adherent Huvec and HeLa cells were fixed by glutaraldehyde and stained with a 0.05% solution of methylene blue for 15 min. After gently washing the stain was eluted by 0.2 mL of 0.33 N HCl in the wells. The optical densities were measured at 660 nm in SUNRISE microplate reader (TECAN).

The $GI_{50}$ and $CC_{50}$ values were defined as being where the concentration-response curve intersected the 50% line, compared to untreated control. These comparisons of the different values were performed with software Magellan (TECAN).

A repeat determination has been conducted in all cases.

All new compounds showed antimicrobial activities. The effect against fungal strains like *Candida albicans, Sporobolomyces salmonicolor, Penicillium notatum, Glomerella* or several *Aspergillus* spp. were higher. Best results could be obtained for deoxyaureonitrile and the halogen-substituted aureothin derivatives with activities comparable to aureothin.

| DOA derivative | $IC_{50}$ |
|---|---|
| R = Ar-CN | 0.5 µg/ml (1 mM) |
| R = Ar-F | 0.5 µg/ml (1 mM) |
| R = Ar-Cl | 0.8 µg/ml (2 mM) |
| R = Ar-Br | 0.5 µg/ml (1 mM) |
| R = Ar-I | 0.5 µg/ml (1 mM) |
| R = Ar-Ph | 8.0 µg/ml (21 mM) |

Biological testings revealed that all new compounds showed not only increased antiproliferative but also cytostatic activities in comparison with aureothin (A). This involves various cell lines like human cervix carcinoma HeLa, mouse fibroblast cells L-929, human umbilical vein endothelial cells Huvec as well as human K-562 leukemia cells. Deoxyaureothin (B) exhibits a 5-fold increased antiproliferative activity against K-562 cells ($IC_{50}$: 5 mM vs. 25 mM of aureothin (A; J. He, M. Müller, C. Hertweck, J. Am. Chem. Soc. 2004, 126, 16742). Strikingly, for deoxyaureonitrile and the halogenated deoxyaureothin derivatives an up to 25-fold increase compared to aureothin could be determined. A wide range was found between maximum effective and ineffective concentrations after incubation for 72 h with all new substrates. The activities were distributed from 1 x $10^{-5}$ to 10 µg $mL^{-1}$. So, in addition to the $IC_{50}$ values the cytostatic activities could be even better demonstrated within the dose-response curves that are characterized by a remarkable flat trend. This offers a broad therapeutic spectrum. In ***Fig. 3*** the differences between aureothin and deoxyaureothin are shown within the K-562 dose-response curves. Those for the respective p-fluoro-analogues as representatives for the new compounds are shown in *Fig.* 4. ***Fig. 5*** and ***Fig. 6*** show the respective cytostatic effects of these compounds on HeLa cells. Due to lower $IC_{50}$ values compared with the aureothin analogues the therapeutic use of deoxyaureothin derivatives could start earlier and involves a flexible growth inhibition of cells. The slow change from toxic to non-toxic

concentrations may be a prerequisite for therapeutic usefulness, where the relatively less toxic part of the curve can be exploited *in vivo* for antineoplastic therapy, with relatively few side effects. In our opinion a combination of both a flat curve and low $IC_{50}$ values would grant useful therapeutic properties.

## Claims

1. An aureothin derivative according to formula (I) or (II):

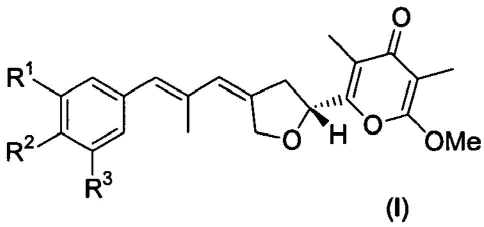

(I)

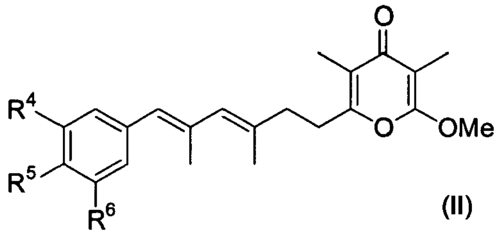

(II)

or a pharmacologically acceptable salt, solvate, hydrate or a pharmacologically acceptable formulation thereof, wherein

$R^1$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^1$ is taken together with $R^2$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^2$ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^2$ is taken together with $R^3$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^3$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes;

$R^4$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^4$ is taken together with $R^5$ to form a 5- to 8-membered carbocyclic or heterocyclic ring that is substituted with

from 0 to 3 substituents which substituents are each independently selected from halogen atom, hydroxy, nitro, amino, alkoxy, carboxyl, alkyl, alkynyl, alkenyl, or aryl;

$R^5$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or

$R^5$ is taken together with $R^6$ to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;

$R^6$ is a hydrogen atom, a halogen atom, a hydroxy, a nitro, a cyano, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes.

**2.** The compound according to claim 1, wherein $R^1$ and accordingly $R^4$ is a hydrogen atom.

**3.** The compound according to claim 1 or claim 2, wherein $R^3$ and accordingly $R^6$ is a hydrogen atom.

**4.** The compound according to any of claims 1 to 3, wherein $R^2$ is a hydrogen atom, halogen atom, methoxy, methyl, or trifluoromethyl.

**5.** The compound according to any of claims 1 to 3, wherein $R^5$ is a hydrogen atom, halogen atom, cyano, nitro, methoxy, or methyl.

**6.** The compound according to claim 1 or claim 3, wherein $R^1$ and $R^2$ and accordingly $R^4$ and $R^5$ are taken together to form a 6-membered carbocyclic ring.

**7.** The compound according to claim 1 or claim 3, wherein $R^1$ and $R^2$ and accordingly $R^4$ and $R^5$ are taken together to form a 6-membered aromatic ring.

**8.** The compound according to claim 1, wherein $R^1$ is a hydrogen atom;
$R^2$ is a hydrogen atom, halogen atom, methoxy, or methyl; and
$R^3$ is a hydrogen atom.

**9.** The compound according to claim 1, wherein $R^4$ is a hydrogen atom;
$R^5$ is a hydrogen atom, halogen atom, cyano, methoxy, or methyl; and
$R^6$ is a hydrogen atom.

**10.** The compound according to claim 1, wherein $R^1$ and accordingly $R^4$ is a hydrogen atom;
$R^2$ and accordingly $R^5$ is a hydrogen atom; and
$R^3$ and accordingly $R^6$ is nitro.

**11.** A pharmaceutical composition that comprises at least one compound according to any one of claims 1 to 10 and, optionally, a carrier substance and/or an adjuvant.

**12.** A method for the preparation of a compound of formula (II), the method comprising the steps of:

(a) fermenting S. *albus* carrying plasmid pMZ01 in the presence of a mutasynthon; and
(b) separating and retaining the compound from the culture broth.

**13.** The method according to claim 12, wherein the mutasynthon is selected from any of

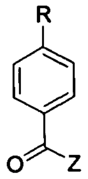 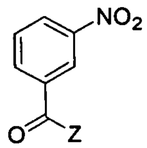 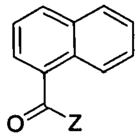

Z= OH or SNAC
R= Me, OMe, F, Cl, Br, I, H, CN

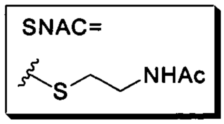

SNAC=

**14.** A method for the preparation of a compound of formula (I), the method comprising the steps of:

(a) fermenting S. *albus* carrying plasmid pHJ110 in the presence of a deoxyaureothin derivative according to formula (II); and
(b) separating and retaining the compound from the culture broth.

**15.** The method according to claim 14, wherein the deoxyaureothin derivative is selected from any of

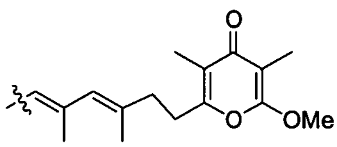

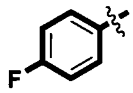 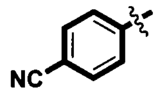 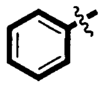

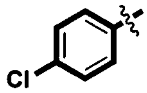 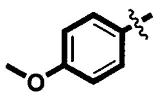 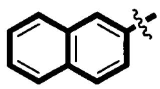

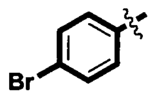 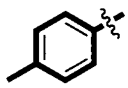

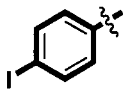 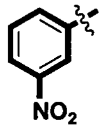

**16.** Compound or pharmaceutical composition according to any one of claims 1 to 12 for the treatment of a bacterial infection, a fungal infection, or cancer.

Fig. 1

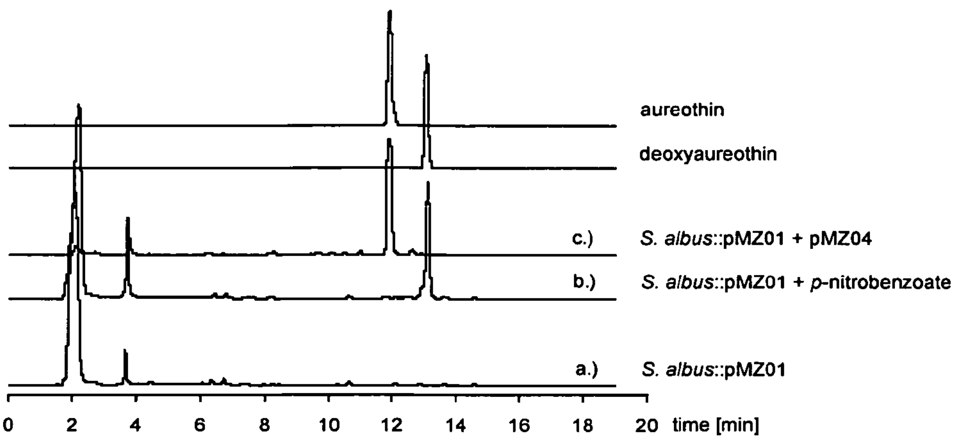
aureothin
deoxyaureothin
c.) S. albus::pMZ01 + pMZ04
b.) S. albus::pMZ01 + p-nitrobenzoate
a.) S. albus::pMZ01
0 2 4 6 8 10 12 14 16 18 20 time [min]

Fig. 2

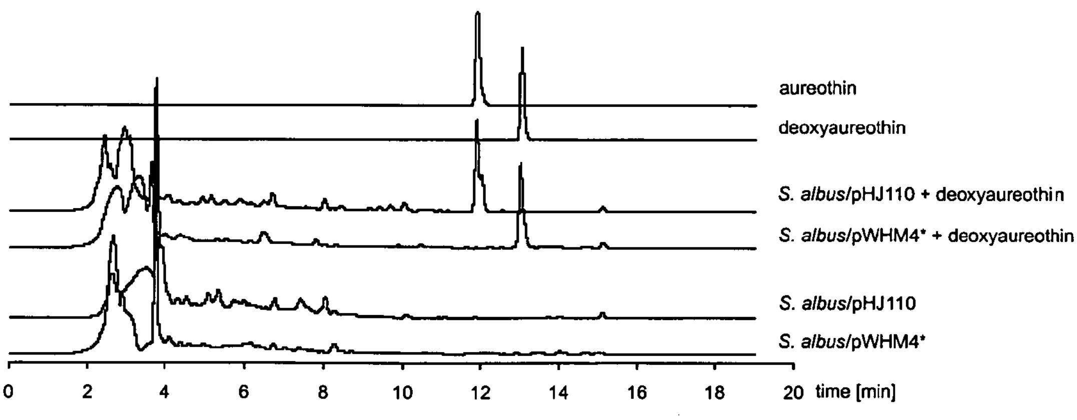
aureothin
deoxyaureothin
S. albus/pHJ110 + deoxyaureothin
S. albus/pWHM4* + deoxyaureothin
S. albus/pHJ110
S. albus/pWHM4*
0 2 4 6 8 10 12 14 16 18 20 time [min]

FIG. 3

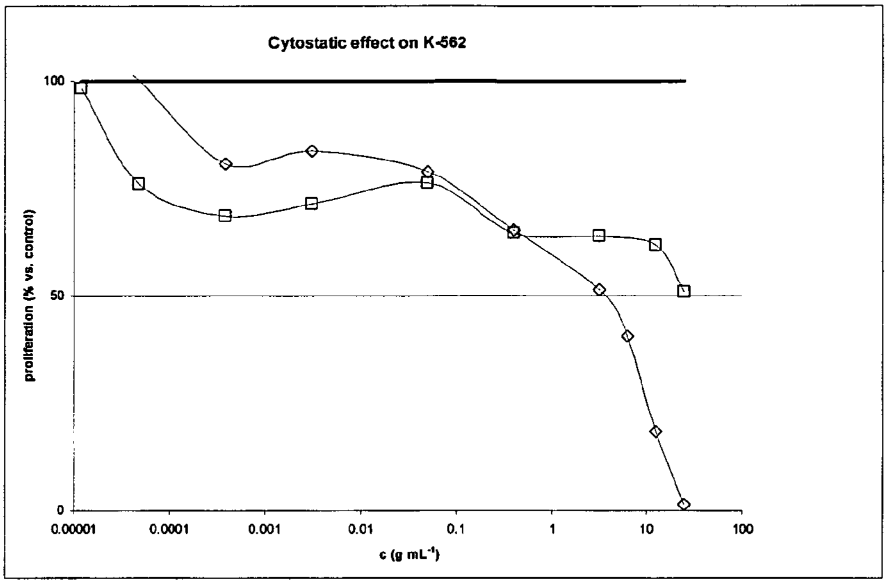


aureothin (□)

deoxyaureothin (◊)

FIG. 4

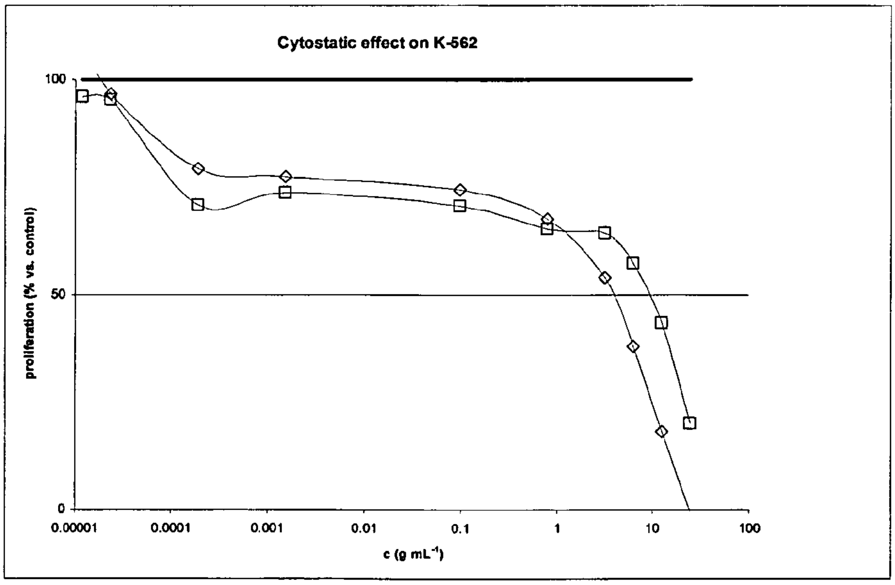


*p*-fluoro-aureothin (□)

*p*-fluoro-deoxyaureothin (◊)

FIG. 5

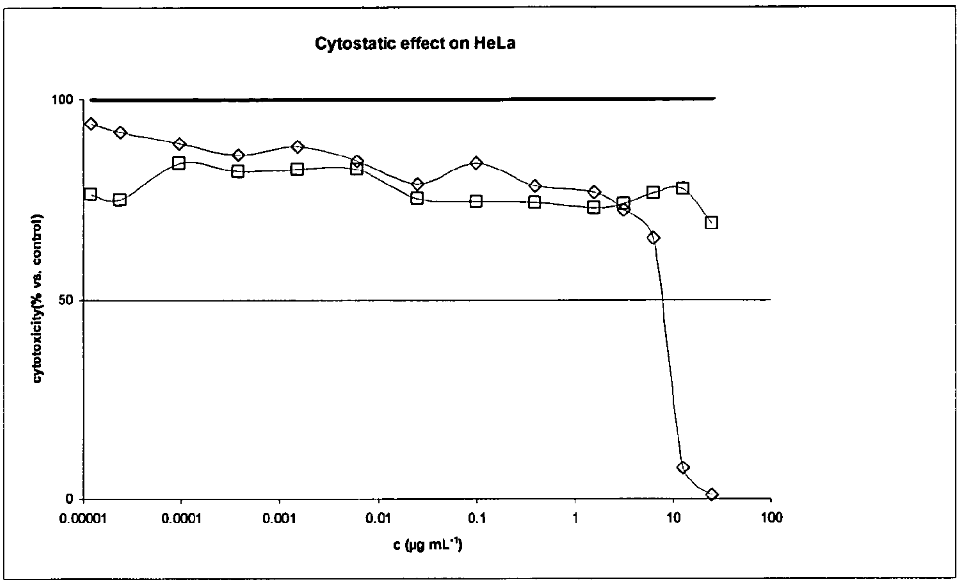


aureothin (□)

deoxyaureothin (◊)

FIG. 6

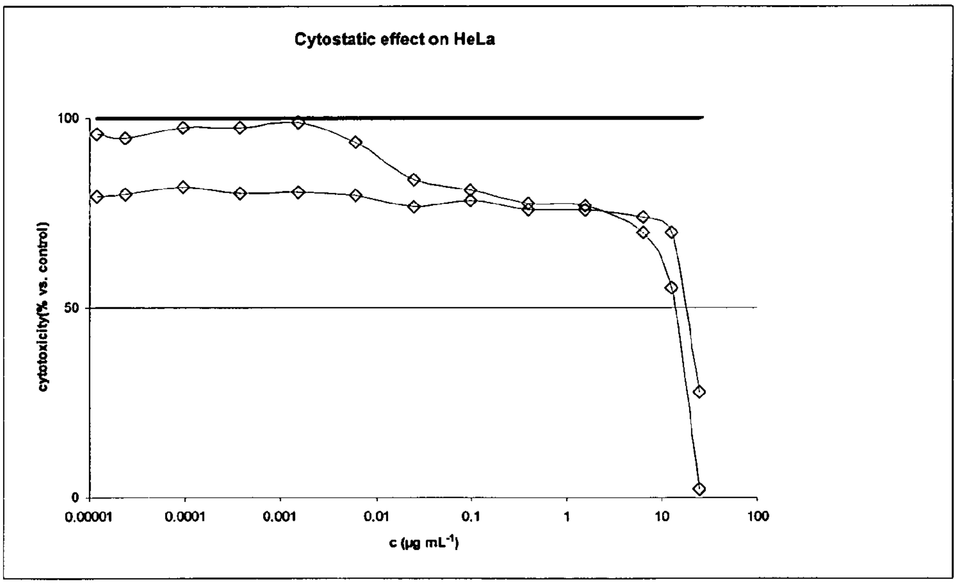


*p*-fluoro-aureothin (□)

*p*-fluoro-deoxyaureothin (◊)

Europäisches Patentamt
European Patent Office
Office européen des brevets

# EUROPEAN SEARCH REPORT

Application Number
EP 08 00 7043

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | JACOBSEN, MIKKEL F. ET AL: "A Short Total Synthesis of Aureothin and N-Acetylaureothamine" ORGANIC LETTERS , 7(4), 641-644 CODEN: ORLEF7; ISSN: 1523-7060, 2005, XP002495062 * the whole document * ----- | 1-3 | INV. C07D407/04 C07D309/38 |
| | | | TECHNICAL FIELDS SEARCHED (IPC) |
| | | | C07D |

~~The present search report has been drawn up for all claims~~

| Place of search | Date of completion of the search | Examiner |
|---|---|---|
| Munich | 9 September 2008 | Bader, Karl Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

Application Number
EP 08 00 7043

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 8, 10-16 (part)

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION SHEET B**

Application Number
EP 08 00 7043

The Search Division considers that the present European patentapplication does not comply with the requirements of unity of invention and relates to severalinventions or groups of inventions, namely:

1. claims: 1-4,8,10-16 (part)

A compound according to formula (I) with R2=H
---

2. claims: 1-4,8,11-16 (part)

A compound according to formula (I) with R2=halogen
---

3. claims: 1-3,11-16 (part)

A compound according to formula (I) with R2=Hydroxy
---

4. claims: 1-3,11-16 (part)

A compound according to formula (I) with R2=amino
---

5. claims: 1-3,11-16 (part)

A compound according to formula (I) with R2=mercapto
---

6. claims: 1-3,11-16 (part)

A compound according to formula (I) with R2=any of the other substituents cited in claim 1
---

7. claims: 1-3,5-7,9-16 (at least in part)

A compound according to claim 1 formula (II)
---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **K. Maeda.** *J. Antibiot. (Tokyo),* 1953, vol. 6, 137
- **Y. Hirata ; H. Nakata ; K. Yamada ; K. Okuhara ; T. Naito.** *Tetrahedron,* 1961, vol. 14, 252
- **J. He ; C. Hertweck.** *Chem. Biol.,* 2003, vol. 10, 1225
- **J. He ; C. Hertweck.** *J. Am. Chem. Soc.,* 2004, vol. 126, 3694
- **R. Winkler ; C. Hertweck.** *ChemBioChem,* 2007, vol. 8, 973
- **G. Zocher ; R. Winkler ; C. Hertweck ; G. E. Schulz.** *J. Mol. Biol.,* 2007
- **J. Staunton ; K. J. Weissman.** *Nat. Prod.,* 2001, vol. 18, 380
- **J. He ; M. Müller ; C. Hertweck.** *J. Am. Chem. Soc,* 2004, vol. 126, 16742
- **M. Müller ; J. He ; C. Hertweck.** *ChemBioChem,* 2006, vol. 7, 37
- Martindale--The Extra Pharmacopoeia. Pharmaceutical Press, 1993
- Remington's Pharmaceutical Sciences
- **F. von Nussbaum ; M. Brands ; B. Hinzen ; S. Weigand ; D. Häbich.** *Angew. Chem.,* 2006, vol. 118, 5194-5254
- *Angew. Chem. Int. Ed.,* 2006, vol. 45, 5072-5129
- **I. Paterson ; E. A. Anderson.** *Science,* 2005, vol. 310, 451-453
- **D. J. Newman ; G. M. Cragg ; K. M. Snader.** *J. Nat. Prod.,* 2003, vol. 66, 1022-1037
- **S. Weist ; R. D. Süssmuth.** *Appl. Microbiol. Biotechnol,* 2005, vol. 68, 141-150
- **K. L. Rinehart.** *Pure Appl. Chem.,* 1977, vol. 49, 1361-1384
- **J. He ; M. Müller ; C. Hertweck.** *J. Am. Chem. Soc.,* 2004, vol. 126, 16742
- **R. V. J. Chari ; B. A. Martell ; J. L. Gross ; S. B. Cook ; S. A. Shah ; W. A. Blättler ; S. J. McKenzie ; V. S. Goldmacher.** *Cancer Res.,* 1992, vol. 52, 127-131
- **K. Okamoto ; K. Harada ; S. Ikeyama ; S. Iwasa.** *Jpn. J. Cancer Res.,* 1992, vol. 83, 761-768
- **C. Liu ; B. M. Tadayoni ; L. A. Bourret ; K. M. Mattocks ; S. M. Derr ; W. C. Widdison ; N. L. Kedersha ; P. D. Ariniello ; V. S. Goldmacher ; J. M. Lambert.** *Proc. Nat. Acad. Sci. USA,* 1996, vol. 93, 8618-8623
- **R. V. Madrigal ; B. W. Zilkowski ; C. R. Smith Jr.** *J. Chem. Ecol.,* 1985, vol. 11, 501-506
- **S. Schlehuber ; A. Skerra.** *Expert Opin Biol Ther.,* 2005, vol. 5, 1453-1462
- **S. Schlehuber ; A. Skerra.** *Drug Discov Today,* 2005, vol. 10, 23-33
- **A. R. Hilgenbrink ; P. S. Low.** *J. Pharm. Sci.,* 2005, vol. 94, 2135-46
- **C. P. Leamon ; J. A. Reddy.** *Adv. Drug Deliv. Rev.,* 2004, vol. 56, 1127-1141
- **P. Dubs ; R. Stüssi.** *Helv. Chim. Acta,* 1976, vol. 59, 1307-1311
- **T. W. Greene ; P. G. M. Wuts.** Protective Groups in Organic Synthesis. John Wiley & Sons, Inc, 1999, 149
- **C. J. Thompson ; J. M. Ward ; D. A. Hoopwood.** *Nature,* 1980, vol. 286, 525
- **K. Ylihonko ; P. Mántsála.** *Microbiol,* 1994, vol. 140, 1359-1365
- **J. Staunton ; A. C. Sutkowski.** *J. Chem. Soc., Chem. Commun.,* 1991, 1110
- **T. Leaf ; L. Cadapan ; C. Carreras ; R. Regentin ; S. Ou ; E. Woo ; G. Ashley ; P. Licari.** *Biotechnol. Prog.,* 2000, vol. 16, 553
- **M. Ziehl ; J. He ; H. M. Dahse ; C. Hertweck.** *Angew. Chem. Int. Ed. Engl.,* 2005, vol. 44, 1202
- **He et al.** *J. Am. Chem. Soc.,* 2004, vol. 126, 16742
- **T. Kieser ; M. J. Bibb ; M. J. Buttner ; K. F. Chater ; D. A. Hopwood.** Practical Streptomyces Genetics. John Innes Foundation, 2000